# EUROPEAN PATENT SPECIFICATION

(11) **EP 1 757 311 B1**
(45) Date of publication and mention of the grant of the patent: **11.02.2009**
(21) Application number: 06017276.4
(22) Date of filing: 22.12.2000
(51) Int. Cl.: A61K 47/48, C07K 7/08, C07K 14/00, C07K 16/22, C07K 16/24, C07K 16/46

(54) **Methods and compositions for prolonging elimination half-times of bioactive compounds**
Verfahren und Zusammensetzungen zur Verlängerung der Entsorgungshalbwertszeit von biowirksamen Verbindungen
Méthodes et compositions pour la prolongation de la demi-période d'élimination de composés bioactifs

(30) Priority: 24.12.1999 US 173048 P
(43) Date of publication of application: 28.02.2007
(62) Divisional of application: 00988373.7
(73) Proprietor: Genentech, Inc., South San Francisco CA 94080-4990 (US)
(72) Inventor: Delano, Warren, San Carlos, CA 94070 (US); Dennis, Mark, S., San Carlos, CA 94070 (US); Lowman, Henry B., El Granada, CA 94018 (US)
(74) Representative: Walton, Seán Malcolm

(56) References cited:
- WO-A-91/01743
- G ZETTLMEISSL ET AL.: "Expression and characterization of human CD4: immunoglobulin fusion proteins" DNA AND CELL BIOLOGY, vol. 9, no. 3, 1990, pages 347-353, XP000941110 US MARY ANN LIEBERT, NEW YORK, NY

## Description

### Field of the Invention

This invention relates to novel, compounds termed peptide ligands which bind IgG-Fc. In particular aspects, the invention relates to compositions comprising a hybrid molecule comprising a peptide ligand domain and an active domain such as a biologically active molecule. The active domain may comprise a molecule useful for diagnostic or therapeutic purposes. In preferred embodiments, the hybrid compositions comprising the peptide ligand domain and active domain have improved pharmacokinetic or pharmacological properties. The invention further provides for the research, diagnostic and therapeutic use of the peptide ligand and includes compositions such as pharmaceutical compositions comprising the peptide ligand molecules.

### Description of Related Disclosures

Phage-display provides a means for generating constrained and unconstrained peptide libraries (Devlin et al., (1990) Science 249:404-406; Cwirla et al., (1990) Proc. Natl. Acad. Sci. USA 87:6378-6382; Lowman (1997) Ann. Rev. Biophys. Biomol. Struct. 26:401-424). These libraries can be used to identify and select peptide ligands that can bind a predetermined target molecule (Lowman (1997), *supra*); Clackson and Wells (1994) Trends Biotechnol. 12:173-184; Devlin *et al.,* (1990) *supra*). The technique has been used to identify peptide motifs that home to a cellular target (Arap et al., (1998) Science 279:377-380); bind the human type I interleukin 1 (IL-1) receptor blocking the binding of IL-α (Yanofsky et al., (1996) Proc. Natl. Acad. Sci. USA 93:7381-7386); bind to and activate the receptor for the cytokine erythropoietin (EPO) (Wrighton et al., (1996) Science 273:458-463); bind the human thrombopoietin receptor and compete with the binding of the natural ligand thrombopoietin (TPO)(Cwirla et al., (1996) Science 276:1696-1699), or to generate affinity improved or matured peptide ligands from native protein binding ligands (Lowman et al., (1991) Biochemistry 30:10832-10838).

Using structurally constrained peptide libraries generated by monovalent phage display, 14 amino acid peptides that specifically bind to insulin-like growth factor 1 binding proteins (IGFBPs) have been isolated (Lowman et al. (1998), Biochemistry 37:8870-8878). The peptides contain a helix structure and bind IGFBPs *in vitro* liberating insulin like growth factor-a (IGF-1) activity (Lowman *et al.,* (1998) *supra*). Utilizing *in vivo* phage selection peptides capable of mediating selective localization to various organs such as brain and kidney (Pasqualini and Ruoslohti (1996) Nature 380:364-366) as well as peptides that home to particular tumor types bearing α_{V}β₃ or αVβ₅ integrins have been identified (Arap et al. (1998), Science 279:377-380). U.S. Patent 5,627,263 describes peptides that are recognized by and selectively bind the α₅β₁ integrin. Examples of affinity or specificity improved proteins include human growth hormone, zinc fingers, protease inhibitors, atrial natriuretic factor, and antibodies (Wells, J. and Lowman H. (1992), Curr. Opin. Struct. Biol. 2:597-604; Clackson, T. and Wells, J. (1994), Trends Biotechnol. 12:173-184; Lowman et al., (1991) Biochemistry 30(10):832.838; Lowman and Wells J. (1993), J. Mol. Biol. 234:564-578; Dennis M. and Lazarus R. (1994), J. Biol. Chem. 269(22):137-144).

It has been suggested that the pharmakodynamics of insulin are altered if bound to serum albumin. Acylation of insulin with saturated fatty acids containing 10-16 carbon atoms produces insulin with affinity for albumin (Kurtzhals, P. et al. (1995) Biochem. J. 312:725-731). Differences in albumin binding affinity among acylated insulins were correlated with the timing of the blood-glucose lowering effects of the various molecules after subcutaneous injection into rabbits. Tighter binding to albumin was correlated with a delay in blood glucose lowering, possibly due to acylated insulin binding albumin in the subcutaneous tissue, resulting in a lower absorption rate of the acylated insulins when compared with non-acylated insulin.

A serum albumin-CD4 conjugate in which the V1 and V2 domains of CD4 were fused with human serum albumin (HSA) has been described (Yeh, P. et al. (1992), Proc. Natl. Acad: Sci. USA 89:1904-1908). The conjugate's elimination half-time was 140-fold that of a soluble CD4 (sCD4) in a rabbit experimental model.

Extended *in vivo* half-times of human soluble complement receptor type 1 (sCR1) fused to the albumin binding domains from *Streptococcal* protein G have been reported (Makrides, S. et al. (1996) J. Pharmacol. Exptl. Ther. 277:532-541). The constructs contained albumin binding domains of protein G having approximately 80 amino acids (fragment BA), and approximately 155 amino acids (fragment BABA).

The pharmacokinetics of a labeled IgG binding domain derived from the Z domain of protein A having approximately 60 amino acids and of a serum albumin binding domain derived from *Streptococcal* protein G (B-domain) having approximately 200 amino acids have been described (EP 0 486,525).

### Summary of the Invention

The present invention provides novel compounds that bind to plasma proteins. The compounds of the present invention (referred to as peptide ligands) are according to claim 1.

Such compounds bind IgG-Fc with an affinity characterized by a dissociation constant, K_{d}, that is less than about 100 µM, preferably less than about 100 nM, and preferably do not substantially bind other plasma proteins. Specific examples of such compounds include linear or cyclic, especially cyclic peptides, preferably between about 10 and 20 amino acid residues in length, and combinations thereof, optionally modified at the N-terminus or C-terminus or both, as well as their salts and derivatives, functional analogues thereof and extended peptide chains carrying amino acids or polypeptides at the termini of the sequences.

In particular aspects the invention is directed to combinations of a peptide ligand with a bioactive compound to form a hybrid molecule that comprises a peptide ligand domain and an active domain. The bioactive compounds of the invention include any compound useful as a therapeutic or diagnostic agent. Non-limiting examples of bioactive compounds include polypeptides such as enzymes, hormones, cytokines, antibodies or antibody fragments, as well as organic compounds such as analgesics, antipyretics, antiinflammatory agents, antibiotics, antiviral agents, anti-fungal drugs, cardiovascular drugs, drugs that affect renal function and electrolyte metabolism, drugs that act on the central nervous system and chemotherapeutic drugs, to name but a few.

In preferred embodiments, the hybrid molecules comprising a peptide ligand domain and an active domain have improved pharmacokinetic or pharmacodynamic properties as compared to the same bioactive molecule comprising the active domain but lacking the peptide ligand domain. The improved pharmacokinetic or pharmacodynamic properties of the hybrids thereby provide for low-dose pharmaceutical formulations and novel pharmaceutical compositions. In certain aspects, the invention provides for methods of using the novel compositions including the therapeutic or diagnostic use of the hybrid molecules.

In particular aspects, the invention is directed to combinations of peptide ligands with bioactive compounds that have relatively short elimination half-times. The combinations are prepared with various objectives in mind, including improving the therapeutic or diagnostic efficacy of the bioactive compound in aspects of the invention involving *in vivo* use of the bioactive compound, by for example, increasing the elimination half-time of the bioactive compound. Fusing or linking (*i.e.*, "conjugating") the peptide ligand directed against a plasma protein such as serum albumin, an immunoglobulin, an apolipoprotein or transferrin to a bioactive compound provides compositions with increased elimination half-times. Such combinations or fusions are conveniently made in recombinant host cells, or by the use of bifunctional crosslinking agents.

Other aspects of the invention include methods and compositions to purify antibodies using peptide ligands having binding affinity for IgG-Fc as disclosed herein.

The present invention further extends to therapeutic and diagnostic application for the compositions described herein. Therefore, the invention includes pharmaceutical compositions comprising a pharmaceutically acceptable excipient and the hybrid molecules of the invention.

### Brief Description of the Drawings

Fig. 1. Phage competitive ELISA assay showing IgG binding of peptide-ligand tagged anti-VEGF Fab-phagemid particles. Four different constructs are shown: pY0192-569 (large filled circles), pY0192-570 (large open circles), PY0317-569 (small filled circles), and pY0317-570 ("x"'s).
Fig.2. BIAcore^{™} analysis of IgG binding to peptide-ligand tagged anti-VEGF Fab Y0317-570 (tagged; top panel) Y0317 Fab (control; bottom panel). A cartoon illustration at top shows a model for the binding events observed in the tagged Fab experiment.
Fig. 3. Group average scrum concentration vs. time data (±SD) are presented in the figure for Fab-Y0317-570 and Fab.Y0317.
Fig. 4. The peptide sequences displayed by phage clones selected for binding to rabbit, human or rat albumin are shown in Figure 4. Also indicated is the ability of individual phage clones to bind the 3 species of immobilized albumin.
Fig. 5A to 5F. Sequences identified following soft randomization are shown in Figure 5 along with their species specificity as determined by phage ELISA.
Fig. 6. Clones originating from the RB soft randomization library were found by ELISA to bind each of these species of albumin and were specific for albumin based upon their lack of binding to ovalbumin and casein.
Fig. 7. Clones that bind to multiple species of albumin (multi-species binders) are listed in Figure 7.
Fig. 8A to 8E. Sequences from libraries selected against rat, rabbit and human albumin are shown in Figures 8A, 8B, and 8C respectively.
Fig. 9. Peptides corresponding to identified phage sequences were synthesized and their affinity for rat, rabbit or mouse albumin measured using the SA08b binding assay.
Fig. 10A peptides corresponding to the SA06 identified phage sequence was synthesized and its affinity for rat, rabbit or mouse albumin measured using the SA08b binding assay.
Fig. 11. The SA06 sequence was added to the carboxy terminus of either the light chain (D3H44-L) or heavy chain (D3H44-Ls) of the Fab. In addition, identical constructs were made with the intra-chain disulfide replaced by alanines (D3H44-Ls and D3H44-Hs, respectively) as depicted in Figure 11.
Fig. 12. Purified D3H44 fusions retained their ability to bind TF as measured using a FX activation assay.
Fig. 13. Purified D3H44 fusions retained their ability to bind TF as measured using a prothrombin time assay that measures prolongation of tissue factor dependent clotting.
Fig. 14 Unlike D3H44 lacking the albumin binding sequence (WT), both D3H44-L and D3H44-Ls are able to bind to albumin as measured in the SA08b binding assay.
Fig. 15 Both D3H44 albumin-binding fusions are capable of binding TF and albumin simultaneously as judged by a biotin-TF binding assay.
Fig. 16 Fusion of the albumin binding peptide to D3H44 results in a protein having improved pharmacokinetic parameters.
Fig. 17. Fusion of the albumin binding peptides to D3H44 results in a protein having improved pharmacokinetic parameters.

### Detailed Description of the Preferred Embodiments

### I. Definitions

The term "peptide ligand" within the context of the present invention is meant to refer to non-naturally occurring amino acid sequences that function to bind a particular target molecule. Peptide ligands within the context of the present invention are generally constrained (that is, having some element of structure as, for example, the presence of amino acids which initiate a β turn or β pleated sheet, or for example, cyclized by the presence of disulfide-bonded Cys residues) or unconstrained (linear) amino acid sequences of less than about 50 amino acid residues, and preferably less than about 40 amino acids residues. Of the peptide ligands less than about 40 amino acid residues, preferred are the peptide ligands of between about 10 and about 30 amino acid residues and especially the peptide ligands of about 20 amino acid residues. However, upon reading the instant disclosure, the skilled artisan will recognize that it is not the length of a particular peptide ligand but its ability to bind a particular target molecule that distinguishes the peptide ligand of the present invention. Therefore peptide ligands of 3, 4, 5, 6, 7, 8, 9, 10, 11, 12, 13, 14, 15, 16, 17, 18, 19, 20, 21, 22, 23, 24 and 25, amino acid residues, for example, are equally likely to be peptide ligands within the context of the present invention.

A peptide ligand of the present invention will bind a target molecule with sufficient affinity and specificity if the peptide ligand "homes" to, "binds" or "targets" a target molecule such as a specific cell type bearing the target molecule *in vitro* and preferably *in vivo* (*see,* for example, the use of the term "homes to," "homing," and "targets" in Pasqualini and Ruoslahti (1996) Nature, 380:364-366 and Arap et al., (1998) Science, 279:377-380). In general, the peptide ligand will bind a target molecule with an affinity characterized by a dissociation constant, K_{d}, of less than about 1 µM, preferably less than about 100 nM and more preferably less than about 10 nM. However, peptide ligands having an affinity for a target molecule of less than about 1 nM and preferably between about 1 pM and 1 nM are equally likely to be peptide ligands within the context of the present invention. In general a peptide ligand that binds a particular target molecule as described above can be isolated and identified by any of a number of art-standard techniques as described herein.

Peptides ligands are amino acid sequences as described above which may contain naturally as well as non-naturally occurring amino acid residues. Therefore, so-called "peptide mimetics" and "peptide analogs" which may include non-amino acid chemical structures that mimic the structure of a particular amino acid or peptide may be peptide ligands within the context of the invention. Such mimetics or analogs are characterized generally as exhibiting similar physical characteristics such as size, charge or hydrophobicity present in the appropriate spacial orientation as found in their peptide counterparts. A specific example of a peptide mimetic compound is a compound in which the amide bond between one or more of the amino acids is replaced by, for example, a carbon-carbon bond or other bond as is well known in the art (*see,* for example Sawyer, in Peptide Based Drug Design pp. 378-422 (ACS, Washington DC 1995)).

Therefore, the term "amino acid" within the scope of the present invention is used in its broadest sense and is meant to include naturally occurring L α-amino acids or residues. The commonly used one and three letter abbreviations for naturally occurring amino acids are used herein (Lehninger, A.L., Biochemistry, 2d ed., pp. 71-92, (1975), Worth Publishers, New York). The correspondence between the standard single letter codes and the standard three letter codes is well known to the skilled artisan, and is reproduced here: A = Ala; C =Cys; D = Asp; E = Glu; F = Phe; G = Gly; H = His; I = Ile; K = Lys; L = Leu; M = Met; N = Asn; P = Pro; Q = Gln; R = Arg; S = Ser; T = Thr; V = Val; W = Trp; Y = Tyr. The term includes D-amino acids as well as chemically modified amino acids such as amino acid analogs, naturally occurring amino acids that are not usually incorporated into proteins such as norleucine, and chemically synthesized compounds having properties known in the art to be characteristic of an amino acid. For example, analogs or mimetics of phenylalanine or proline, which allow the same conformational restriction of the peptide compounds as natural Phe or Pro are included within the definition of amino acid. Such analogs and mimetics are referred to herein as "functional equivalents" of an amino acid. Other examples of amino acids are listed by Roberts and Vellaccio The Peptides: Analysis, Synthesis, Biology, Gross and Meiehofer, eds., Vol. 5 p. 341, Academic Press, Inc., N.Y. 1993, which is incorporated herein by reference.

Peptide ligands synthesized by, for example, standard solid phase synthesis techniques, are not limited to amino acids encoded by genes. Commonly encountered amino acids which are not encoded by the genetic code, include, for example, those described in International Publication No. WO 90/01940 such as, for example, 2-amino adipic acid (Aad) for Glu and Asp; 2-aminopimelic acid (Apm) for Glu and Asp; 2-aminobutyric (Abu) acid for Met, Leu, and other aliphatic amino acids; 2-aminoheptanoic acid (Ahe) for Met, Leu and other aliphatic amino acids; 2-aminoisobutyric acid (Aib) for Gly; cyclohexylalanine (Cha) for Val, and Leu and Ile; homoarginine (Har) for Arg and Lys; 2,3-diaminopropionic acid (Dpr) for Lys, Arg and His; N-ethylglycine (EtGly) for Gly, Pro, and Ala; N-ethylglycine (EtGly) for Gly, Pro, and Ala; N-ethylasparigine (EtAsn) for Asn, and Gln; Hydroxyllysine (Hyl) for Lys; allohydroxyllysine (AHyl) for Lys; 3-(and 4)-hydoxyproline (3Hyp, 4Hyp) for Pro, Ser, and Thr; allo-isoleucine (AIle) for Ile, Leu, and Val; ρ-amidinophenylalanine for Ala; N-methylglycine (MeGly, sarcosine) for Gly, Pro, and Ala; N-methylisoleucine (Melle) for Ile; Norvaline (Nva) for Met and other aliphatic amino acids; Norleucine (Nle) for Met and other aliphatic amino acids; Ornithine (Orn) for Lys, Arg and His; Citrulline (Cit) and methionine sulfoxide (MSO) for Thr, Asn and Gln; N-methylphenylalanine (MePhe), trimethylphenylalanine, halo (F, Cl, Br, and I) phenylalanine, trifluorylphenylalanine, for Phe.

Peptide ligands within the context of the present invention may be "engineered", *i.e.*, they are non-native or non-naturally occurring peptide ligands. By "non-native" or "non-naturally occurring" is meant that the amino acid sequence of the particular peptide ligand is not found in nature. That is to say, amino acid sequences of non-native or non-naturally occurring peptide ligands do not correspond to an amino acid sequence of a naturally occurring protein or polypeptide. Peptide ligands of this variety may be produced or selected using a variety of techniques well known to the skilled artisan. For example, constrained or unconstrained peptide libraries may be randomly generated and displayed on phage utilizing art standard techniques, for example, Lowman et al., (1998) Biochemistry 37:8870-8878.

Peptide ligands, when used within the context of the present invention, may be "conjugated" to a therapeutic or diagnostic substance. The term "conjugated" is used in its broadest sense to encompass all methods of attachment or joining that are known in the art. For example, in a typical embodiment, the therapeutic or diagnostic substance is a protein (referred to herein as a "protein therapeutic"), and the peptide ligand will be an amino acid extension of the Cor N-terminus of the protein therapeutic. In addition, a short amino acid linker sequence may lie between the protein therapeutic and the peptide ligand. In this scenario, the peptide ligand, optional linker and protein therapeutic will be coded for by a nucleic acid comprising a sequence encoding protein therapeutic operably linked to (in the sense that the DNA sequences are contiguous and in reading frame) an optional linker sequence encoding a short polypeptide as described below, and a sequence encoding the peptide ligand. In this typical scenario, the peptide ligand is considered to be "conjugated" to the protein therapeutic optionally via a linker sequence. In a related embodiment, the peptide ligand amino acid sequence may interrupt or replace a section of the protein therapeutic amino acid sequence, provided, of course, that the insertion of the peptide ligand amino acid sequence does not interfere with the function of the protein therapeutic. In this embodiment, the "conjugate" may be coded for by a nucleic acid comprising a sequence encoding protein therapeutic interrupted by and operably linked to a sequence encoding the peptide ligand. In a further typical embodiment, the peptide will be linked, *e.g.*, by chemical conjugation to the protein therapeutic or other therapeutic optionally via a linker sequence. Typically, according to this embodiment, the peptide ligand will be linked to the protein therapeutic via a side chain of an amino acid somewhere in the middle of the protein therapeutic that doesn't interfere with the therapeutic's activity. Here again, the peptide is considered to be "conjugated" to the therapeutic.

As used within the context of the present invention the term "target molecule" includes, proteins, peptides, glycoproteins, glycopeptides, glycolipids, polysaccharides, oligosaccharides, nucleic acids, and the like. Target molecules include, for example, extracellular molecules such as various serum factors including but not limited to plasma proteins such as serum albumin, immunoglobulins, apolipoproteins or transferrin, or proteins found on the surface of erythrocytes or lymphocytes, provided, of course, that binding of the peptide ligand to the cell surface protein does not substantially interfere with the normal function of the cell. "Antibodies" and "immunoglobulins" are usually heterotetrameric glycoproteins of about 150,000 Daltons, composed of two identical light (L) chains and two identical heavy (H) chains.

Papain digestion of antibodies produces two identical antigen-binding fragments, called "Fab" fragments or regions, each with a single antigen-binding site, and a residual "Fc" fragment or region. Although-the boundaries of the Fc region of an immunoglobulin heavy chain might vary, the human IgG. heavy chain Fc region is usually defined to stretch from an amino acid residue at position Cys226, or from Pro230, to the carboxyl-terminus thereof.

Pepsin treatment yields an F(ab')₂ fragment that has two antigen-combining sites and is still capable of cross-linking antigen. The Fab' fragment contains the constant domain of the light chain and the first constant domain (CH1) of the heavy chain.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, cats, cows, *etc.* Preferably, the mammal is human.

A "disorder" is any conditions that would benefit from treatment with the compositions comprising the peptide ligands of the invention. This includes chronic and acute disorders or diseases including those pathological conditions which predispose the mammal to the disorder in question.

"Elimination half-time" is used in its ordinary sense, as is described in Goodman and Gillman's The Pharmaceutical Basis of Therapeutics 21-25 (Alfred Goodman Gilman, Louis S. Goodman, and Alfred Gilman, eds., 6th ed. 1980). Briefly, the term is meant to encompass a quantitative measure of the time course of drug elimination. The elimination of most drugs is exponential (*i.e.*, follows first-order kinetics), since drug concentrations usually do not approach those required for saturation of the elimination process. The rate of an exponential process may be expressed by its rate constant, k, which expresses the fractional change per unit of time, or by its half-time, t½, the time required for 50% completion of the process. The units of these two constants are time⁻¹ and time, respectively. A first-order rate constant and the half-time of the reaction are simply related (k x t½ = 0.693) and may be interchanged accordingly. Since first-order elimination kinetics dictates that a constant fraction of drug is lost per unit time, a plot of the log of drug concentration versus time is linear at all times following the initial distribution phase (*i.e.* after drug absorption and distribution are complete). The half-time for drug elimination can be accurately determined from such a graph.

"Transfection" refers to the taking up of an expression vector by a host cell whether or not any coding sequences are in fact expressed. Numerous methods of transfection are known to the ordinarily skilled artisan, for example, CaPO₄ precipitation and electroporation. Successful transfection is generally recognized when any indication of the operation of this vector occurs within the host cell.

"Transformation" means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described in section 1.82 of Sambrook et al., Molecular Cloning (2nd ed.), Cold Spring Harbor Laboratory, NY (1989), is generally used for prokaryotes or other cells that contain substantial cell-wall barriers. Infection with*Agrobacterium tumefaciens* is used for transformation of certain plant cells, as described by Shaw et al., (1983) Gene, 23:315 and WO 89/05859 published 29 June 1989. For mammalian cells without such cell walls, the calcium phosphate precipitation method described in sections 16.30-16.37 of Sambrook *et al., supra,* is preferred. General aspects of mammalian cell host system transformations have been described by Axel in U.S. Patent No. 4,399,216 issued 16 August 1983. Transformations into yeast are typically carried out according to the method of Van Solingen et al., (1977) J. Bact., 130:946 and Hsiao et al., (1979) Proc. Natl. Acad. Sci. (USA), 76:3829. However, other methods for introducing DNA into cells such as by nuclear injection, electroporation, or by protoplast fusion may also be used.

As used herein, the term "pulmonary administration" refers to administration of a formulation of the invention through the lungs by inhalation. As used, herein, the term "inhalation" refers to intake of air to the alveoli. In specific examples, intake can occur by self-administration of a formulation of the invention while inhaling, or by administration via a respirator, *e.g.*, to an patient on a respirator. The term "inhalation" used with respect to a formulation of the invention is synonymous with "pulmonary administration."

As used herein, the term "parenteral" refers to introduction of a compound of the invention into the body by other than the intestines, and in particular, intravenous (i.v.), intraarterial (i.a.), intraperitoneal (i.p.), intramuscular (i.m.), intraventricular, and subcutaneous (s.c.) routes.

As used herein, the term "aerosol" refers to suspension in the air. In particular, aerosol refers to the particlization of a formulation of the invention and its suspension in the air. According to the present invention, an aerosol formulation is a formulation comprising a compound of the present invention that is suitable for aerosolization, *i.e.*, particlization and suspension in the air, for inhalation or pulmonary administration.

### II. Modes for Carrying out the Invention

### A. Peptide Ligands

Peptide ligands within the context of the present invention bind a target, IgG-Fc, and can be identified in a direct binding assay, or by their ability to compete for target binding with a known ligand for the target.

Peptide ligands of the present invention bind IgG-Fc and can be identified by their ability to compete for binding of IgG-Fc in an *in vitro* assay with a peptide ligand having the general formula:
Xaaᵢ-Cys-Xaaⱼ-Cys-Xaaₖ (SEQ ID NO: 1), wherein Xaaᵢ is absent or is a peptide of between 1 and 4 amino acids, preferably 4 amino acids; Xⱼ is preferably 9 amino acids having a preferred sequence Xaa-Xaa-Xaa-Xaa-Xaa-Xaa-Leu-Val-Trp (SEQ ID NO: 10); or Xaa-Xaa-Xaa-Xaa-Gly-Glu-Leu-Val-Trp (SEQ ID NO: 11); or Xaa₁-Xaa₂-Xaa₃-Xaa₄-Gly-Glu-Leu-Val-Trp (SEQ ID NO: 12), wherein Xaa₁ is Ala, Ser, or Thr; Xaa₂ is Trp or Tyr; Xaa₃ is His, or Trp; Xaa₄ is Leu or Met, and Xaaₖ is absent or between 1 and 5 amino acids, preferably 5 amino acids, so long as the cyclic peptide or analog thereof retains the qualitative biological activity of binding IgG-Fc described above.
Preferred among this group of compounds are compounds comprising the sequence:

Xaa-Xaa-Xaa-Xaa-Cys-Xaa-Xaa-Xaa-Xaa-Xaa-Xaa-Leu-Val-Trp-Cys-Xaa-Xaa-Xaa-
Xaa-Xaa (SEQ ID NO: 13);

Xaa-Xaa-Xaa-Xaa-Cys-Xaa-Xaa-Xaa-Xaa-Gly-Glu-Leu-Val-Trp-Cys-Xaa-Xaa-Xaa-Xaa-
Xaa (SEQ ID NO: 14);

   Xaa₁-Xaa₂-Xaa₃-Xaa₄-Cys-Xaa₅-Xaa₆-Xaa₇-Xaa₈-Gly-Glu-Leu-Val-Trp-Cys-Xaa₉-Xaa₁₀-Xaa₁₁-Xaa₁₂-Xaa₁₃ (SEQ ID NO: 15), wherein Xaa₅ is Ala, Ser, or Thr; Xaa₆ is Trp or Tyr; Xaa₇ is His, or Trp; and Xaa₈ is Leu or Met; and
   Xaa₁-Xaa₂-Xaa₃-Xaa₄-Cys-Xaa₅-Xaa₆-Xaa₇-Xaa₈-Gly-Glu-Leu-Val-Trp-Cys-Xaa₉-Xaa₁₀-Xaa₁₁-Xaa₁₂-Xaa₁₃ (SEQ ID NO: 16) wherein Xaa₄ is Ser, Arg, or Asp; Xaa₅ is Ala, Ser, or Thr; Xaa₆ is Trp, Tyr; Xaa₇ is His, or Trp; Xaa₈ is Leu or Met; and Xaa₉ is Glu, Ser, Thr or Val. In particular embodiments, the IgG-Fc binding peptide ligands of the present invention will compete with any of the peptide ligands represented in SEQ ID.NO: 3 - SEQ ID NO: 4, SEQ ID NO: 9; and SEQ ID NO: 13 - SEQ ID NO: 111 described herein and preferably will compete with SEQ ID NO: 9 for binding IgG-Fc.

As will be appreciated from the foregoing, the term "compete" and "ability to compete" are relative terms. Thus the terms, when used to describe the peptide ligands of the present invention, refer to peptide ligands that produce a 50% inhibition of binding of, for example SEQ ID NO: 9 or SEQ ID NO: z4, when present at 50 µM, preferably when present at 1 µM, more preferably 100 nM, and preferably when present at 1 nM or less in a standard competition assay as described herein. Such peptide ligands generally will bind IgG-Fc with an affinity of less than 1 µM, preferably less than about 100 nM and more preferably less than about 10 nM as determined by a standard competition assay such as the one described in the Example sections. However, peptide ligands having an affinity for IgG-Fc of less than about 1 nM and preferably between about 1 pM and 1 nM are equally likely to be peptide ligands within the context of the present invention.

For *in vitro* assay systems to determine whether a peptide or other compound has the "ability" to compete with a peptide ligand for binding to an IgG-Fc as noted herein, the skilled artisan can employ any of a number of standard competition assays. Competitive binding assays rely on the ability of a labeled standard to compete with the test sample analyte for binding with a limited amount of ligand. The amount of analyte in the test sample is inversely proportional to the amount of standard that becomes bond to the ligand.

Thus, the skilled artisan may determine whether a peptide or other compound has the ability to compete with a peptide ligand for binding to an IgG-Fc employing procedures which include but are not limited to competitive assay systems using techniques such as radioimmunoassays (RIA), enzyme immunoassays (EIA), preferably the enzyme linked immunosorbent assay (ELISA), "sandwich" immunoassays, immunoradiometric assays, fluorescent immunoassays, and immunoelectrophoresis assays, to name but a few.

For these purposes the selected peptide ligand will be labeled with a detectable moiety (the detectably labeled peptide ligand hereafter called the "tracer") and used in a competition assay with a candidate compound for binding IgG-Fc domain or other target. Numerous detectable labels are available which can be preferably grouped into the following categories:
(a) Radioisotopes, such as ³⁵S, ¹⁴C, ¹²⁵I, ³H, and ¹³¹I. The peptide compound can be labeled with the radioisotope using the techniques described in Coligen et al., eds., Current Protocols in Immunology, Volumes 1 and 2 (1991), Wiley-Interscience, New York, N.Y., for example and radioactivity can be measured using scintillation counting.
(b) Fluorescent labels such as rare earth chelates (europium chelates) or fluorescein and its derivatives, rhodamine and its derivatives, dansyl, lissamine, phycoerythrin and Texas Red are available. The fluorescent labels can be conjugated to the peptide compounds using the techniques disclosed in Current Protocols in Immunology, *supra,* for example. Fluorescence can be quantified using a fluorimeter.
(c) Various enzyme-substrate labels are available and U.S. Patent No. 4,275,149 provides a review of some of these. The enzyme preferably catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRP), alkaline phosphatase, ϑ-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example:
(i) Horseradish peroxidase (HRP) with hydrogen peroxidase as a substrate, wherein the hydrogen peroxidase oxidizes a dye precursor (*e.g.* ABTS, orthophenylene diamine (OPD) or 3,3',5,5'-tetramethyl benzidine hydrochloride (TMB));
(ii) alkaline phosphatase (AP) with para-nitrophenyl phosphate as chromogenic substrate; and
(iii) β-D-galactosidase (β-D-Gal) with a chromogenic substrate (*e.g.* p-nitrophenyl-β-D-galactosidase) or fluorogenic substrate 4-methylumbelliferyl-β-D-galactosidase.

According to a particular assay, the tracer is incubated with immobilized target in the presence of varying concentrations of unlabeled candidate compound. Increasing concentrations of successful candidate compound effectively compete with binding of the tracer to immobilized target. The concentration of unlabeled candidate compound at which 50% of the maximally-bound tracer is displaced is referred to as the "IC₅₀" and reflects the IgG binding affinity of the candidate compound. Therefore a candidate compound with an IC₅₀ of 1 mM displays a substantially weaker interaction with the target than a candidate compound with an IC₅₀ of 1 µM.

In some phage display ELISA assays, binding affinity of a mutated ("mut") sequence was directly compared of a control ("con") peptide using methods described in B.C. Cunningham, D.G. Lowe, B. Li, B.D. Bennett, and J.A. Wells, EMBO J. 13:2508 (1994) and characterized by the parameter EC₅₀. Assays were performed under conditions where EC₅₀(con)/EC₅₀(mut) will approximate K_{d}(con)/K_{d}(mut).

Accordingly, the invention provides compounds "having the ability to compete" for target molecules such as IgG or human serum albumin binding in an *in vitro* assay as described. Preferably the compound has an IC₅₀ for the target such as IgG or human serum albumin of less than 1 µM. Preferred among these compound are compounds having an IC₅₀ of less than about 100 nM and preferably less than about 10 nM or less than about 1 nM. In further preferred embodiments according to this aspect of the invention the compounds display an IC₅₀ for the target molecule such as IgG or human serum albumin of less than about 100 pM and more preferably less than about 10 pM.

A preferred *in vitro* assay for the determination of a candidate compound's ability to compete with a peptide ligand described herein is as follows and is described more fully in the Examples. In preferred embodiments the candidate compound is a peptide. The ability of a candidate compound to compete with a labeled peptide ligand tracer for binding to IgG or human serum albumin is monitored using an ELISA. Dilutions of a candidate compound in buffer are added to microtiter plates coated with IgG or human serum albumin (as described in the Example Sections) along with tracer for 1 hr. The microtiter plate is washed with wash buffer and the amount of tracer bound to IgG or human serum albumin measured.

### B. Peptide Ligand Combinations

According to the present invention, the peptide ligand is optionally linked to a bioactive compound to form a hybrid molecule that comprises a peptide ligand domain and an active domain. The bioactive compounds of the invention include any compound useful as a therapeutic or diagnostic agent. Non-limiting examples of bioactive compounds include polypeptides such as enzymes, hormones, cytokines, antibodies or antibody fragments, as well as organic compounds such as analgesics, antipyretics, antiinflammatory agents, antibiotics, antiviral agents, anti-fungal drugs, cardiovascular drugs, drugs that affect renal function and electrolyte metabolism, drugs that act on the central nervous system, chemotherapeutic drugs, etc. According to the present invention the peptide ligand domain is joined to an active domain, optionally via a flexible linker domain.

The hybrid molecules of the present invention are constructed by combining a peptide ligand domain with a suitable active domain. Depending on the type of linkage and its method of production, the peptide ligand domain may be joined via its N- or C-terminus to the N- or C-terminus of the active domain. For example, when preparing the hybrid molecules of the present invention via recombinant techniques, nucleic acid encoding a peptide ligand will be operably linked to nucleic acid encoding the active domain sequence, optionally via a linker domain. Typically the construct encodes a fusion protein wherein the C-terminus of the peptide ligand is joined to the N-terminus of the active domain. However, especially when synthetic techniques are employed, fusions where, for example, the N-terminus of the peptide ligand is joined to the N- or C-terminus of the active domain also are possible. In some instances, the peptide ligand domain may be inserted within the active domain molecule rather than being joined to the active domain at its N-or C-terminus. This configuration may be used to practice the invention so long as the functions of the peptide ligand domain and the active domain are preserved. For example, a peptide ligand may be inserted into a non-binding light chain CDR of an immunoglobulin without interfering with the ability of the immunoglobulin to bind to its target. Regions of active domain molecules that can accommodate peptide ligand domain insertions may be identified empirically (*i.e*., by selecting an insertion site, randomly, and assaying the resulting conjugate for the function of the active domain), or by sequence comparisons amongst a family of related active domain molecules (*e.g*., for active domains that are proteins) to locate regions of low sequence homology. Low sequence homology regions are more likely to tolerate insertions of peptide ligands domains than are regions that are well-conserved. For active domain molecules whose three-dimensional structures are known (*e.g*. from X-ray crystallographic or NMR studies), the three-dimensional structure may provide guidance as to peptide ligand insertion sites. For example, loops or regions with high mobility (*i.e.*, large temperature or "B" factors) are more likely to accommodate peptide ligand domain insertions than are highly ordered regions of the structure, or regions involved in ligand binding or catalysis.

### C. Linker Domains

According to the present invention, the peptide ligand domain is optionally linked to the active domain via a linker. The linker component of the hybrid molecule of the invention does not necessarily participate in but may contribute to the function of the hybrid molecule. Therefore, according to the present invention, the linker domain, is any group of molecules that provides a spatial bridge between the active domain and the peptide ligand domain.

The linker domain can be of variable length and makeup, however, according to the present invention, it is the length of the linker domain and not its structure that is important. The linker domain preferably allows for the peptide ligand domain of the hybrid molecule to bind, substantially free of steric and/or conformational restrictions to the target molecule. Therefore, the length of the linker domain is dependent upon the character of the two "functional" domains of the hybrid molecule, *i.e*., the peptide ligand domain and the active domain.

One skilled in the art will recognize that various combinations of atoms provide for variable length molecules based upon known distances between various bonds (Morrison, and Boyd, Organic Chemistry, 3rd Ed, Allyn and Bacon, Inc., Boston, MA (1977)). For example, the linker domain may be a polypeptide of variable length. The amino acid composition of the polypeptide determines the character and length of the linker. In a preferred embodiment, the linker molecule comprises a flexible, hydrophilic polypeptide chain. Exemplary, linker domains comprises one or more Gly and or Ser residues, such as those described in the Example sections herein.

### D. Recombinant Synthesis

The present invention encompasses a composition of matter comprising an isolated nucleic acid, preferably DNA, encoding a peptide ligand or a hybrid molecule comprising a peptide ligand domain and a polypeptide active domain as described herein. DNAs encoding the peptides of the invention can be prepared by a variety of methods known in the art. These methods include, but are not limited to, chemical synthesis by any of the methods described in Engels et al. (1989), Agnew. Chem. Int. Ed. Engl. 28:716-734, such as the triester, phosphite, phosphoramidite and H-phosphonate methods. In one embodiment, codons preferred by the expression host cell are used in the design of the encoding DNA. Alternatively, DNA encoding the peptides of the invention can be altered to encode one or more variants by using recombinant DNA techniques, such as site specific mutagenesis (Kunkel et al. (1991), Methods Enzymol., 204:125-139; Carter et al. (1986), Nucl. Acids Res. 13:4331; Zoller et al. (1982), Nucl. Acids Res. 10:6487), cassette mutagenesis (Wells et al. (1985), Gene 34:315), restriction selection mutagenesis (Carter, Directed Mutagenesis: A Practical Approach (M.J. McPherson, ed.) IRL Press, Oxford, 1991), and the like.

According to preferred aspects described above, the nucleic acid encodes a peptide ligand capable of binding IgG-Fc.

According to another preferred aspect of the invention, the nucleic acid encodes a hybrid molecule comprising a peptide ligand domain sequence and an active domain. In this aspect of the invention, the active domain may comprise any polypeptide compound useful as a therapeutic or diagnostic agent, *e.g*., enzymes, hormones, cytokines, antibodies or antibody fragments. The nucleic acid molecule according to this aspect of the present invention encodes a hybrid molecule and the nucleic acid encoding the peptide ligand domain sequence is operably linked to (in the sense that the DNA sequences are contiguous and in reading frame) the nucleic acid encoding the biologically active agent. Optionally these DNA sequences may be linked through a nucleic acid sequence encoding a linker domain amino acid sequence.

According to this aspect, the invention further comprises an expression control sequence operably linked to the DNA molecule encoding a peptide of the invention, an expression vector, such as a plasmid, comprising the DNA molecule, wherein the control sequence is recognized by a host cell transformed with the vector, and a host cell transformed with the vector. In general, plasmid vectors contain replication and control sequences which are derived from species compatible with the host cell. The vector ordinarily carries a replication site, as well as sequences which encode proteins that are capable of providing phenotypic selection in transformed cells.

For expression in prokaryotic hosts, suitable vectors include pBR322 (ATCC No. 37,017), phGH107 (ATCC No. 40,011), pBO475, pS0132, pRIT5, any vector in the pRIT20 or pRIT30 series (Nilsson and Abrahmsen (1990), Meth. Enzymol. 185:144-161), pRIT2T, pKK233-2, pDR540 and pPL-lambda. Prokaryotic host cells containing the expression vectors of the present invention include *E*. *coli* K12 strain 294 (ATCC NO. 31,446), *E*. *coli* strain JM101 (Messing et al.(1981), Nuc/. Acid Res. 9:309), *E. coli* strain B, *E*. *coli* strain _1776 (ATCC No. 31537), *E*. *coli* c600, *E. coli* W3110 (F-, gamma-, prototrophic, ATCC No. 27,325), *E. coli* strain 27C7 (W3110, *tonA, phoA E15, (argF-lac)169, ptr3, degP41, ompT, kan*^{r}) (U.S. Patent No. 5,288,931, ATCC No. 55,244), *Bacillus subtilis, Salmonella typhimurium, Serratia marcesans,* and *Pseudomonas* species.

In addition to prokaryotes, eukaryotic organisms, such as yeasts, or cells derived from multicellular organisms can be used as host cells. For expression in yeast host cells, such as common baker's yeast or *Saccharomyces cerevisiae,* suitable vectors include episomally-replicating vectors based on the 2-micron plasmid, integration vectors, and yeast artificial chromosome (YAC) vectors. For expression in insect host cells, such as Sf9 cells, suitable vectors include baculoviral vectors. For expression in plant host cells, particularly dicotyledonous plant hosts, such as tobacco, suitable expression vectors include vectors derived from the Ti plasmid of *Agrobacterium tumefaciens.*

Examples of useful mammalian host cells include monkey kidney CV1 line transformed by SV40 (COS-7, ATCC CRL 1651); human embryonic kidney line (293 or 293 cells subcloned for growth in suspension culture, Graham et al. (1977), J. Gen Virol. 36:59); baby hamster kidney cells (BHK, ATCC CCL 10); Chinese hamster ovary cells/-DHFR (CHO, Urlaub and Chasin (1980), Proc. Natl. Acad. Sci. USA 77:4216); mouse sertoli cells (TM4, Mather (1980), Biol. Reprod. 23:243-251); monkey kidney cells (CV1 ATCC CCL.70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W 138, ATCC CCL 75); human liver cells (Hep G2, HB 8065); mouse mammary tumor (MMT 060562, ATCC CCL51); TRI cells (Mather et al. (1982), Annals N.Y. Acad. Sci. 383:44-68); MRC 5 cells; FS4 cells; and a human hepatoma cell line (Hep G2). For expression in mammalian host cells, useful vectors include vectors derived from SV40, vectors derived from cytomegalovirus such as the pRK vectors, including pRK5 and pRK7 (Suva et al. (1987), Science 237:893-896; EP 307,247 (3/15/89), EP 278,776 (8/17/88)) vectors derived from vaccinia viruses or other pox viruses, and retroviral vectors such as vectors derived from Moloney's murine leukemia virus (MoMLV).

Optionally, the DNA encoding the peptide of interest is operably linked to a secretory leader sequence resulting in secretion of the expression product by the host cell into the culture medium. Examples of secretory leader sequences include STII, ecotin, lamB, herpes GD, 1pp, alkaline phosphatase, invertase, and alpha factor. Also suitable for use herein is the 36 amino acid leader sequence of protein A (Abrahmsen et al. (1985), EMBO J. 4:3901).

Host cells are transfected and preferably transformed with the above-described expression or cloning vectors of this invention and cultured in conventional nutrient media modified as appropriate for inducing promoters, selecting transformants, or amplifying the genes encoding the desired sequences.

Prokaryotic host cells used to produce the present peptides can be cultured as described generally in Sambrook *et al., supra.*

The mammalian host cells used to produce peptides of the invention can be cultured in a variety of media. Commercially available media such as Ham's F10 (Sigma), Minimal Essential Medium ((MEM), Sigma), RPMI-1640 (Sigma), and Dulbecco's Modified Eagle's Medium ((DMEM), Sigma) are suitable for culturing the host cells. In addition, any of the media described in Ham and Wallace (1979), Meth. in Enz. 58:44, Barnes and Sato (1980), Anal. Biochem. 102:255, U.S. Patent Nos. 4,767,704; 4,657,866; 4,927,762; or 4,560,655; WO 90/03430; WO 87/00195; U.S. Pat. Re. 30,985; or U.S. 5,122,469, the disclosures of all of which are incorporated herein by reference, may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The host cells referred to in this disclosure encompass cells in *in vitro* culture as well as cells that are within a host animal.

### E. Chemical Synthesis

Another method of producing the compounds of the invention involves chemical synthesis. This can be accomplished by using methodologies well known in the art (*see* Kelley, R.F. & Winkler, M.E. in Genetic Engineering Principles and Methods, Setlow, J.K, ed., Plenum Press, N.Y., Vol. 12, pp 1-19 (1990); Stewart, J.M. Young, J.D., Solid Phase Peptide Synthesis, Pierce Chemical Co., Rockford, IL (1984); *see also* U.S. Pat. Nos. 4,105,603; 3,972,859; 3,842,067; and 3,862,925).

Peptide ligands of the invention can be prepared conveniently using solid-phase peptide synthesis. Merrifield (1964), J. Am. Chem. Soc. 85:2149; Houghten (1985), Proc. Natl. Acad. Sci. USA 82:5132. Solid-phase peptide synthesis also can be used to prepare the hybrid molecule compositions of the invention if the active domain is or comprises a polypeptide.

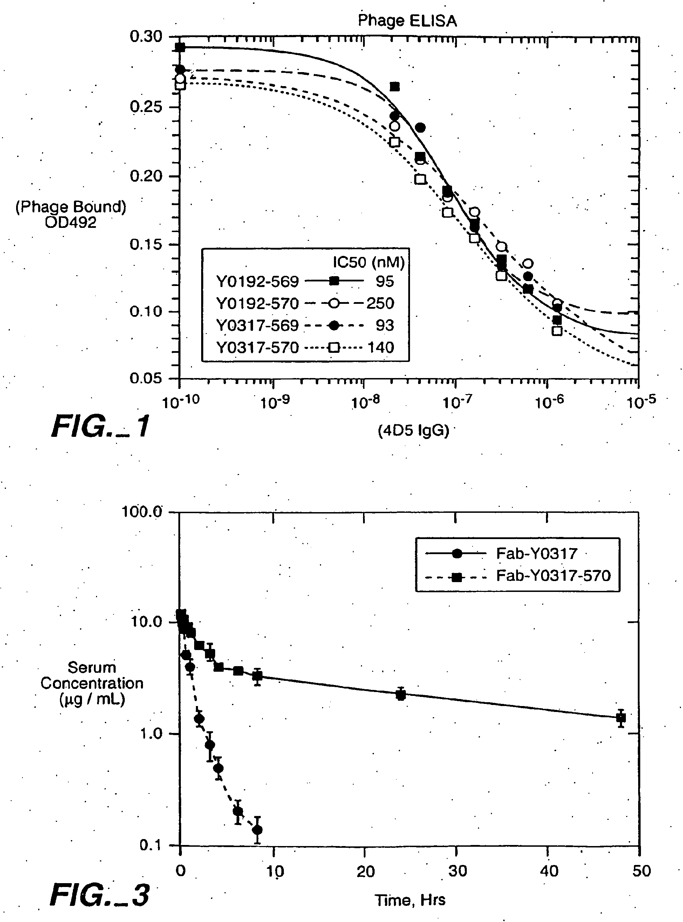

Solid-phase synthesis begins at the carboxy terminus of the nascent peptide by coupling a protected amino acid to an inert solid support. The inert solid support can be any macromolecule capable of serving as an anchor for the C-terminus of the initial amino acid. Typically, the macromolecular support is a cross-linked polymeric resin (*e.g*., a polyamide or polystyrene resin) as shown in Figures 1-1 and 1-2, on pages 2 and 4 of Stewart and Young, *supra.* In one embodiment, the C-terminal amino acid is coupled to a polystyrene resin to form a benzyl ester. A macromolecular support is selected such that the peptide anchor link is stable under the conditions used to deprotect the α-amino group of the blocked amino acids in peptide synthesis. If a base-labile α-protecting group is used, then it is desirable to use an acid-labile link between the peptide and the solid support. For example, an acid-labile ether resin is effective for base-labile Fmoc-amino acid peptide synthesis as described on page 16 of Stewart and Young, *supra.* Alternatively, a peptide anchor link and α-protecting group that are differentially labile to acidolysis can be used. For example, an aminomethyl resin such as the phenylacetamidomethyl (Pam) resin works well in conjunction with Boc-amino acid peptide synthesis as described on pages 11-12 of Stewart and Young, *supra.*

After the initial amino acid is coupled to an inert solid support, the α-amino protecting group of the initial amino acid is removed with, for example, trifluoroacetic acid (TFA) in methylene chloride and neutralized in, for example, triethylamine (TEA). Following deprotection of the initial amino acid's α-amino group, the next α-amino and side chain protected amino acid in the synthesis is added. The remaining α-amino and, if necessary, side chain protected amino acids are then coupled sequentially in the desired order by condensation to obtain an intermediate compound connected to the solid support. Alternatively, some amino acids may be coupled to one another to form a fragment of the desired peptide followed by addition of the peptide fragment to the growing solid phase peptide chain.

The condensation reaction between two amino acids, or an amino acid and a peptide, or a peptide and a peptide can be carried out according to the usual condensation methods such as the axide method, mixed acid anhydride method, DCC (N,N'-dicyclohexylcarbodiimide) or DIC (N,N'-diisopropylcarbodiimide) methods, active ester method, p-nitrophenyl ester method, BOP (benzotriazole-1-yl-oxy-tris [dimethylamino] phosphonium hexafluorophosphate) method, N-hydroxysuccinic acid imido ester method, *etc*., and Woodward reagent K method.

It is common in the chemical synthesis of peptides to protect any reactive side chain groups of the amino acids with suitable protecting groups. Ultimately, these protecting groups are removed after the desired polypeptide chain has been sequentially assembled. Also common is the protection of the α-amino group on an amino acid or peptide fragment while the C-terminal carboxy group of the amino acid or peptide fragment reacts with the free N-terminal amino group of the growing solid phase polypeptide chain, followed by the selective removal of the α-amino group to permit the addition of the next amino acid or peptide fragment to the solid phase polypeptide chain. Accordingly, it is common in polypeptide synthesis that an intermediate compound is produced which contains each of the amino acid residues located in the desired sequence in the peptide chain wherein individual residues still carry side-chain protecting groups. These protecting groups can be removed substantially at the same time to produce the desired polypeptide product following removal from the solid phase.

α- and ε-amino side chains can be protected with benzyloxycarbonyl (abbreviated Z), isonicotinyloxycarbonyl (iNOC), o-chlorobenzyloxycarbonyl [Z(2Cl)], p-nitrobenzyloxycarbonyl [Z(NO₂)], p-methoxybenzyloxycarbonyl [Z(OMe)], t-butoxycarbonyl (Boc), t-amyloxycarbonyl (Aoc), isobornyloxycarbonyl, adamantyloxycarbonyl, 2-(4-biphenyl)-2-propyloxycarbonyl (Bpoc), 9-fluorenylmethoxycarbonyl (Fmoc), methylsulfonyethoxycarbonyl (Msc), trifluoroacetyl, phthalyl, formyl, 2-nitrophenylsulphenyl (NPS), diphenylphosphinothioyl (Ppt), and dimethylphosphinothioyl (Mpt) groups, and the like.

Protective groups for the carboxy functional group are exemplified by benzyl ester (OBzl), cyclohexyl ester (Chx), 4-nitrobenzyl ester (ONb), t-butyl ester (Obut), 4-pyridylmethyl ester (OPic), and the like. It is often desirable that specific amino acids such as arginine, cysteine, and serine possessing a functional group other than amino and carboxyl groups are protected by a suitable protective group. For example, the guanidino group of arginine may be protected with nitro, p-toluenesulfonyl, benzyloxycarbonyl, adamantyloxycarbonyl, p-methoxybenzesulfonyl, 4-methoxy-2,6-dimethylbenzenesulfonyl (Nds), 1,3,5-trimethylphenysulfonyl (Mts), and the like. The thiol group of cysteine can be protected with p-methoxybenzyl, trityl, and the like.

Many of the blocked amino acids described above can be obtained from commercial sources such as Novabiochem (San Diego, CA), Bachem CA (Torrence, CA) or Peninsula Labs (Belmont, CA).

Stewart and Young, *supra,* provides detailed information regarding procedures for preparing peptides. Protection of α-amino groups is described on pages 14-18, and side chain blockage is described on pages 18-28. A table of protecting groups for amine, hydroxyl and sulfhydryl functions is provided on pages 149-151.

After the desired amino acid sequence has been completed, the peptide can be cleaved away from the solid support, recovered and purified. The peptide is removed from the solid support by a reagent capable of disrupting the peptide-solid phase link, and optionally deprotects blocked side chain functional groups on the peptide. In one embodiment, the peptide is cleaved away from the solid phase by acidolysis with liquid hydrofluoric acid (HF), which also removes any remaining side chain protective groups. Preferably, in order to avoid alkylation of residues in the peptide (for example, alkylation of methionine, cysteine, and tyrosine residues), the acidolysis reaction mixture contains thio-cresol and cresol scavengers. Following HF cleavage, the resin is washed with ether, and the free peptide is extracted from the solid phase with sequential washes of acetic acid solutions. The combined washes are lyophilized, and the peptide is purified.

### F. Chemical Conjugation of Hybrids

In certain embodiments of the present invention, the hybrid molecules may comprise active domains that are organic compounds having diagnostic or therapeutic utility, or alternatively, fusions between a peptide ligand domain and a polypeptide active domain in configurations that cannot be encoded in a single nucleic acid. Examples of the latter embodiment include fusions between the amino terminus of a peptide ligand and the amino terminus of the active domain, or fusions between the carboxy-terminus of a peptide ligand and the carboxy-terminus of the active domain.

Chemical conjugation may be employed to prepare these embodiments of the hybrid molecule, using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene, 2,6-diisocyanate), and bis-active fluorine compounds (such as 1,5-difluoro-2,4-dinitrobenzene).

### G. Disulfide-Linked Peptides

As described above, some embodiments of the invention include cyclized peptide ligands. Peptide ligands may be cyclized by formation of a disulfide bond between cysteine residues. Such peptides can be made by chemical synthesis as described above and then cyclized by any convenient method used in the formation of disulfide linkages. For example, peptides can be recovered from solid phase synthesis with sulfhydryls in reduced form, dissolved in a dilute solution wherein the intramolecular cysteine concentration exceeds the intermolecular cysteine concentration in order to optimize intramolecular disulfide bond formation, such as a peptide concentration of 25 mM to 1 µM, and preferably 500 µM to 1 µM, and more preferably 25 µM to 1 µM, and then oxidized by exposing the free sulfhydryl groups to a mild oxidizing agent that is sufficient to generate intramolecular disulfide bonds, e.g., molecular oxygen with or without catalysts such as metal cations, potassium ferricyanide, sodium tetrathionate, *etc.* Alternatively, the peptides can be cyclized as described in Pelton et al. (1986), J. Med. Chem. 29:2370-2375.

Cyclization can be achieved by the formation, for example, of a disulfide bond or a lactam bond between a first Cys and a second Cys. Residues capable of forming a disulfide bond include, for example, Cys, Pen, Mpr, and Mpp and its 2-amino group-containing equivalents. Residues capable of forming a lactam bridge include, for example, Asp Glu, Lys, Orn, αβ-diaminobutyric acid, diaminoacetic acid, aminobenzoic acid and mercaptobenzoic acid. The compounds herein can be cyclized for example via a lactam bond which can utilize the side chain group of a non-adjacent residue to form a covalent attachment to the N-terminus amino group of Cys or other amino acid. Alternative bridge structures also can be used to cyclize the compounds of the invention, including for example, peptides and peptidomimetics, which can cyclize via S-S, CH2-S, CH2-O-CH2, lactam ester or other linkages.

### H. Pharmaceutical Compositions

Pharmaceutical compositions which comprise the hybrid molecules of the invention may be administered in any suitable manner, including parental, topical, oral, or local (such as aerosol or transdermal) or any combination thereof.

Other suitable compositions of the present invention comprise any of the above-noted compositions with a pharmaceutically acceptable carrier, the nature of the carrier differing with the mode of administration, for example, in oral administration, usually using a solid carrier and ini.v. administration, a liquid salt solution carrier.

The compositions of the present invention include pharmaceutically acceptable components that are compatible with the subject and the protein of the invention. These generally include suspensions, solutions and elixirs, and most especially biological buffers, such as phosphate buffered saline, saline, Dulbecco's Media, and the like. Aerosols may also be used, or carriers such as starches, sugars, microcrystalline cellulose, diluents, granulating agents, lubricants, binders, disintegrating agents, and the like (in the case of oral solid preparations, such as powders, capsules, and tablets).

As used herein, the term "pharmaceutically acceptable" generally means approved by a regulatory agency of the Federal or a state government or listed in the U.S. Pharmacopeia or other generally recognized pharmacopeia for use in animals, and more particularly in humans.

The formulation of choice can be accomplished using a variety of the aforementioned buffers, or even excipients including, for example, pharmaceutical grades of mannitol, lactose, starch, magnesium stearate, sodium saccharin cellulose, magnesium carbonate, and the like. "PEGylation" of the compositions may be achieved using techniques known to the art (*see* for example International Patent Publication No. WO92/16555, U.S. Patent No. 5,122,614 to Enzon, and International Patent Publication No. WO92/00748).

A preferred route of administration of the present invention is in the aerosol or inhaled form. The compounds of the present invention, combined with a dispersing agent, or dispersant, can be administered in an aerosol formulation as a dry powder or in a solution or suspension with a diluent.

As used herein, the term "dispersant" refers to a agent that assists aerosolization of the compound or absorption of the protein in lung tissue, or both. Preferably the dispersant is pharmaceutically acceptable. Suitable dispersing agents are well known in the art, and include but are not limited to surfactants and the like. For example, surfactants that are generally used in the art to reduce surface induced aggregation of a compound, especially a peptide compound, caused by atomization of the solution forming the liquid aerosol, may be used. Nonlimiting examples of such surfactants are surfactants such as polyoxyethylene fatty acid esters and alcohols, and polyoxyethylene sorbitan fatty acid esters. Amounts of surfactants used will vary, being generally within the range of from about 0.001% to about 4% by weight of the formulation. In a specific aspect, the surfactant is polyoxyethylene sorbitan monooleate or sorbitan trioleate. Suitable surfactants are well known in the art, and can be selected on the basis of desired properties, depending on the specific formulation, concentration of the compound, diluent (in a liquid formulation) or form of powder (in a dry powder formulation), *etc.*

Moreover, depending on the choice of the peptide ligand, the desired therapeutic effect, the quality of the lung tissue (*e.g*., diseased or healthy lungs), and numerous other factors, the liquid or dry formulations can comprise additional components, as discussed further below.

The liquid aerosol formulations generally contain the peptide ligand/active domain hybrid and a dispersing agent in a physiologically acceptable diluent. The dry powder aerosol formulations of the present invention consist of a finely divided solid form of the peptide ligand/active domain hybrid and a dispersing agent. With either the liquid or dry powder aerosol formulation, the formulation must be aerosolized. That is, it must be broken down into liquid or solid particles in order to ensure that the aerosolized dose actually reaches the alveoli. In general the mass median dynamic diameter will be 5 micrometers or less in order to ensure that the drug particles reach the lung alveoli (Wearley, L.L. (1991), Crit. Rev. in Ther. Drug Carrier Systems 8:333). The term "aerosol particle" is used herein to describe the liquid or solid particle suitable for pulmonary administration, i.e., that will reach the alveoli. Other considerations such as construction of the delivery device, additional components in the formulation and particle characteristics are important. These aspects of pulmonary administration of a drug are well known in the art, and manipulation of formulations, aerosolization means and construction of a delivery device require at most routine experimentation by one of ordinary skill in the art.

With regard to construction of the delivery device, any form of aerosolization known in the art, including but not limited to nebulization, atomization or pump aerosolization of a liquid formulation, and aerosolization of a dry powder formulation, can be used in the practice of the invention. A delivery device that is uniquely designed for administration of solid formulations is envisioned. Often, the aerosolization of a liquid or a dry powder formulation will require a propellant. The propellant may be any propellant generally used in the art. Specific nonlimiting examples of such useful propellants are a chloroflourocarbon, a hydrofluorocarbon, a hydochlorofluorocarbon, or a hydrocarbon, including triflouromethane, dichlorodiflouromethane, dichlorotetrafuoroethanol, and 1,1,1,2-tetraflouroethane, or combinations thereof.

In a preferred aspect of the invention, the device for aerosolization is a metered dose inhaler. A metered dose inhaler provides a specific dosage when administered, rather than a variable dose depending on administration. Such a metered dose inhaler can be used with either a liquid or a dry powder aerosol formulation. Metered dose inhalers are well known in the art.

Once the peptide ligand/active domain hybrid reaches the lung, a number of formulation-dependent factors affect the drug absorption. It will be appreciated that in treating a disease or disorder that requires circulatory levels of the compound, such factors as aerosol particle size, aerosol particle shape, the presence or absence of infection, lung disease or emboli may affect the absorption of the compounds. For each of the formulations described herein, certain lubricators, absorption enhancers, protein stabilizers or suspending agents may be appropriate. The choice of these additional agents will vary depending on the goal. It will be appreciated that in instances where local delivery of the compounds is desired or sought, such variables as absorption enhancement will be less critical.

### I. Liquid Aerosol Formulations

The liquid aerosol formulations of the present invention will typically be used with a nebulizer. The nebulizer can be either compressed air driven or ultrasonic. Any nebulizer known in the art can be used in conjunction with the present invention such as but not limited to: Ultravent, Mallinckrodt, Inc. (St. Louis, MO), the Acorn II nebulizer (Marquest Medical Products, Englewood CO). Other nebulizers useful in conjunction with the present invention are described in U.S. Patent Nos. 4,624,251 issued November 25, 1986; 3,703,173 issued November 21, 1972; 3,561,444 issued February 9, 1971 and 4,635,627 issued January 13, 1971.

The formulation may include a carrier. The carrier is a macromolecule which is soluble in the circulatory system and which is physiologically acceptable where physiological acceptance means that those of skill in the art would accept injection of said carrier into a patient as part of a therapeutic regime. The carrier preferably is relatively stable in the circulatory system with an acceptable elimination half-time. Such macromolecules include but are not limited to soya lecithin, oleic acid and sorbetan trioleate, with sorbitan trioleate preferred.

The formulations of the present embodiment may also include other agents useful for protein stabilization or for the regulation of osmotic pressure. Examples of the agents include but are not limited to salts, such as sodium chloride, or potassium chloride, and carbohydrates, such as glucose, galactose or mannose, and the like.

### J. Aerosol Dry Powder Formulations

It is also contemplated that the present pharmaceutical formulation will be used as a dry powder inhaler formulation comprising a finely divided powder form of the peptide ligand and a dispersant. The form of the compound will generally be a lyophilized powder. Lyophilized forms of peptide ligand/active domain hybrid compounds can be obtained through standard techniques.

In another embodiment, the dry powder formulation will comprise a finely divided dry powder containing one or more compounds of the present invention, a dispersing agent and also a bulking agent. Bulking agents useful in conjunction with the present formulation include such agents as lactose, sorbitol, sucrose, or mannitol, in amounts that facilitate the dispersal of the powder from the device.

### K. Research, Manufacturing, and Diagnostic Compositions

In a preferred embodiment, the peptide ligands or the hybrid molecules of the invention are non-covalently adsorbed or covalently bound to a macromolecule, such as a solid support. It will be appreciated that the invention encompasses macromolecules complexed with the peptide ligands or hybrid molecules. The peptide ligands of the invention are directed against IgG-Fc. Such peptide ligands may be used as affinity purification agents. In this process, the peptide ligands are immobilized on a solid phase support such as a Sephadex resin or filter paper, using methods well known in the art. The immobilized peptide ligand is contacted with a sample containing the immunoglobulin protein (or fragment thereof) to be purified, and thereafter the support is washed with a suitable solvent that will remove substantially all the material in the sample except the immunoglobulin protein, which is bound to the immobilized peptide ligand. Finally, the support is washed with another suitable solvent, such as glycine buffer, pH 5.0, that will release the immunoglobulin protein from the peptide ligand.

In general, the solid support is an inert matrix, such as a polymeric gel, comprising a three-dimensional structure, lattice or network of a material. Almost any macromolecule, synthetic or natural, can form a gel in a suitable liquid when suitably cross-linked with a bifunctional reagent. Preferably, the macromolecule selected is convenient for use in affinity chromatography. Most chromatographic matrices used for affinity chromatography are xerogels. Such gels shrink on drying to a compact solid comprising only the gel matrix. When the dried xerogel is resuspended in the liquid, the gel matrix imbibes liquid, swells and returns to the gel state. Xerogels suitable for use herein include polymeric gels, such as cellulose, cross-linked dextrans (e.g. Sepharose), agarose; cross-linked agarose, polyacrylamide gels, and polyacrylamide-agarose gels.

Alternatively, aerogels can be used for affinity chromatography. These gels do not shrink on drying but merely allow penetration of the surrounding air. When the dry gel is exposed to liquid, the latter displaces the air in the gel. Aerogels suitable for use herein include porous glass and ceramic gels.

Also encompassed herein are the peptide ligands or hybrid molecules of the invention coupled to derivatized gels wherein the derivative moieties facilitate the coupling of the hybrid molecules to the gel matrix and avoid steric hindrance of the peptide ligand-target molecule interaction in affinity chromatography. Alternatively, spacer arms can be interposed between the gel matrix and the hybrid molecules for similar benefits.

A variation on the above contemplates the use of gene fusions and the use of the peptide ligands as purification reagents. According to this aspect of the invention the gene encoding a peptide ligand is associated, in a vector, with a gene encoding another protein or a fragment of another protein. This results in the peptide ligand being produced by the host cell as a fusion with another protein or peptide. The "other" protein or peptide is often a protein or peptide which can be secreted by the cell, making it possible to isolate and purify the other protein from the culture medium and eliminating the necessity of destroying the host cells which arises when the other protein remains inside the cell. Alternatively, the fusion protein can be expressed intracellularly. It is useful to use fusion proteins that are highly expressed.

The use of gene fusions is analogous to the use of Protein A fusions which are often used because the binding of protein A, or more specifically the Z domain of protein A binds to IgG and provides an "affinity handle" for the purification of the fused protein. According to a preferred aspect of the invention, peptide ligands which bind serum albumin are use as "affinity handles" for the purification of fused proteins on a solid serum albumin support. For example, a DNA sequence encoding the desired peptide ligand can be fused by site directed mutagenesis to the gene for protein. After expression and secretion, the fusion protein can be purified on a matix of serum albumin to which the peptide ligand will bind. After purification the peptide ligand can be enzymatically or chemically cleaved to yield free protein or left intact to aid in increasing the elimination half life of the fused protein. Fusion proteins can be cleaved using chemicals, such as cyanogen bromide, which cleaves at a methionine, or hydroxylamine, which cleaves between an Asn and Gly residue. Using standard recombinant DNA methodology, the nucleotide base pairs encoding these amino acids may be inserted just prior to the 5' end of the gene encoding the desired peptide. Alternatively, one can employ proteolytic cleavage of fusion protein. Carter, in Protein Purification: From Molecular Mechanisms to Large-Scale Processes, Ladisch et al., eds. (American Chemical Society Symposium Series No. 427, 1990), Ch 13, pages 181-193.

The following examples are offered by way of illustration and not by way of limitation.

The invention is directed to IgG-Fc peptide ligands other examples illustrate principles and methods used by the inventors.

### EXAMPLE 1

### IgG-Fc Peptide Ligands

An *in vitro* selection designed to identify peptide ligands which bind the IgG-Fc surface without the constraint that the peptides function *in vivo* was performed. The selection was accomplished using a combination of polyvalent and monovalent phage display which has recently been applied to generate peptides that bind a variety of cellular hormones and receptors. N. C. Wrighton, et al. (1996), Science 273:458, O. Livnah, et al. (1996), Science 273:464. A single disulfide-constrained peptide library was constructed that consisted of 4x10⁹ different peptides of the form Xaaᵢ-Cys-Xaaⱼ-Cys-Xaaₖ (SEQ ID NO: 1) wherein Xaa is a random amino acid from an NNS codon, i+j+k = 18, and j = 4 through 10. This library was expressed on the surface of M13 bacteriophage as an N-terminal fusion to the gene VIII protein with a short linker consisting of glycine and serine residues. H. B. Lowman et al. (1998), Biochemistry 37: 8870-8878. More particularly, the library construct contained an STII secretion signal peptide, the peptide library of twenty amino acid length, *i.e*., Xaaᵢ-Cys-Xaaⱼ-Cys-Xaaₖ (SEQ ID NO: 1) wherein Xaa is a random amino acid from an NNS codon, i+j+k = 18, and j = 4 through 10, a Gly-Gly-Gly-Ser-Gly-Gly-Gly linker (SEQ ID NO: 2), and the M13 gene VIII starting at the first residue of the mature protein.

In principle, peptides could be selected that bind to potentially any region of the IgG-Fc due to the unbiased nature of this library. However, after several rounds of selection, the library became dominated by a single peptide, Fc-I (Glu-Thr-Gln-Arg-Cys-Thr-Trp-His-Met-Gly-Glu-Leu-Val-Trp-Cys-Glu-Arg-Glu-His-Asn) (SEQ ID NO: 3). Selections were performed as described in H. B. Lowman, *et al., supra,* with the following modifications: microtiter wells were coated using 5 µg/ml IgG-Fc; Casein Blocker Buffer (Pierce) was used in place of 0.1% BSA to better prevent non-specific binding; elution of phage was effected with either 75 mM DTT or 0.2 mM glycine pH 2.0 with equivalent results. IgG-Fc was obtained by papain cleavage of CD4-IgG₁ immunoadhesin protein, Capon et al. (1989), Nature, 337: 525. Cleaved material was purified over Protein A Sepharose followed by Superdex-75 (Pharmacia) and then quantified by absorbance at 280 nm.

Repetition of the selection experiment again gave Fc-I and also a related peptide, Fc-II (Lys-Glu-Ala-Ser-Cys-Ser-Tyr-Trp-Leu-Gly-Glu-Leu-Val-Trp-Cys-Val-Ala-Gly-Val-Glu) (SEQ ID NO: 4). The Fc-II peptide shared the cysteine spacing and the internal Gly-Glu-Leu-Val-Trp (SEQ ID NO: 134) sequence seen in Fc-I. Apparently, these two peptides bound IgG-Fc with an affinity high enough to be selected over any of the other IgG-Fc binding peptides present in the starting pool. Both peptides were synthesized on solid phase using standard 9-fluorenylmethoxycarbonyl protocols and purified by reversed-phase HPLC. Masses were confirmed by electrospray mass spectrometry, and purified peptides were quantified by UV absorbance at 280 nm.

Competition ELISAs were performed in a manner similar to the method described in H. B. Lowman, *et al., supra.* Briefly, Protein A Z-domain was immobilized on microtiter wells at a concentration of 5 µg/ml, blocked, and washed as described. A matrix of mixtures ofbiotinylated-IgG-Fc at concentrations from 312 nM to 0.3 nM and peptide at concentrations from 215 µM to 0.8 nM was prepared. These mixtures were incubated with immobilized Protein A Z-domain for 1 hour. Plates were then washed and developed as described using avidin/HRP conjugate. Inhibition curves were then computed for each concentration of biotin-IgG-Fc, and then the curve of half-maximal inhibition, "IC₅₀", was extrapolated to zero biotin-IgG-Fc concentration in order to obtain a Kᵢ. The Fc-I and Fc-II peptides both were found to compete with Protein A (Z-domain) (B. Nilsson et al. (1987), Protein Eng. 1:107) for binding to IgG-Fc with inhibition constants (Kᵢ) of about 5 µM. The results imply that these peptides bind to an overlapping site on IgG-Fc that coincides with the Protein A binding site.

The DNA sequences of the Fc-II peptide was moved to a monovalent phage display format by cassette mutagenesis to give a construct with the STII signal sequence, the Fc-II peptide Lys-Glu-Ala-Ser-Cys-Ser-Tyr-Trp-Leu-Gly-Glu-Leu-Val-Trp-Cys-Val-Ala-Gly-Val-Glu (SEQ ID NO: 4), a Gly-Gly-Gly-Pro-Gly-Gly-Gly linker (SEQ ID NO: 5), and the M13 gene III protein starting at residue 253. The Fc-II sequence was affinity-matured by monovalent phage display. Five residue blocks were randomly mutated in six separate libraries to exhaustively cover the non-cysteine positions in the peptide sequence and then screened against IgG-Fc.

A series of second generation monovalent phage display libraries were constructed based on the Fc-II sequence Lys-Glu-Ala-Ser-Cys-Ser-Tyr-Trp-Leu-Gly-Glu-Leu-Val-Trp-Cys-Val-Ala-Gly-Val-Glu (SEQ ID NO: 4) in which five sequential residues were randomized using NNS codons in each library starting at positions 1, 4, 7,10, 12, and 16, excluding the two cysteines. Each library had a diversity of approximately 1x10⁸. These libraries were independently screened for binding to IgG-Fc for six rounds and then sequenced. Preferred residues from this selection were then recombined using three additional libraries that spanned the entire peptide sequence. The three additional libraries were constructed using the degeneracy of the genetic code to recombine the preferred amino acids at each position into one peptide. The DNA sequences for these libraries contained the following mixtures of bases (IUPAC codes): DRG GWA GMA RRC TGC KCT TRS CAC MTG GGC GAG CTG GTC TGG TGC RVC RVM BKC GAS KDW (SEQ ID NO: 6), DRS VWG SVG RRC TGC KCC TRS YRS MTG GGC GAG CTG GTC TGG TGC RNC VVS NBS GWS KDM (SEQ ID NO: 7), and DNS NNS NNS VNS TGC BVG TDS HRS MDS GGC GAG STC KKG WRG TGC RNM NNS NNS NNS NNM (SEQ ID NO: 8). These libraries also were sorted against IgG-Fc for six rounds and then sequenced.

After screening against IgG-Fc, the consensus patterns from these libraries suggested a highly conserved 13-residue core sequence (Asp-Cys-Ala-Trp-His-Leu-Gly-Glu-Leu-Val-Trp-Cys-Thr) (SEQ ID NO: 9). The corresponding peptide (Fc-III) was synthesized and found to inhibit binding of Protein A (Z-domain) to Fc with an IC₅₀ of 100 nM. Thus, although Fc-III is seven residues shorter than Pc-II, it binds 50-times more tightly. Despite its smaller size, the binding affinity of Fc-III to Fc was only ten-fold weaker than that of the domains from Protein A and Protein G, which are each about four times larger and bind with K_{d}S around 10 nM. S. R. Fahnestock, et al. in Bacterial Immunoglobulin-Binding Proteins (Academic Press, Inc. 1990) Vol. 1, chap. 11. R. Karlsson, L. Jendeberg, B. Nilsson, J. Nilsson, P. Nygren (1995), J. Immuno. Methods 183:43.

Table 1 lists the amino acid sequences and IgG-Fc binding affinities of exemplary IgG-Fc peptide ligands that were identified using the procedures described above.

**Table I**

| **IgG-Fc Peptide Ligand Sequences and Affinities** | | |
|---|---|---|
| **Sequence** | **Sequences ID NO** | **Binding Affinity** |
| **Peptides** *All peptides have an N-terminal amine and a C-terminal amide | | |
| | | |
| KEASCSYWLGELVWCVAGVE | SEQ ID NO: 4 | 5000 nM (Kᵢ) |
| ETQRCTWHMGEL VWCEREHN | SEQ ID NO: 3 | 5000 nM (Kᵢ) |
| DLADCSWHMGELVWCSRVEG | SEQ ID NO: 17 | 50 nM (K_{d}) |
| WEADCAWHLGELVWCTPMEF | SEQ ID NO: 18 | 30 nM (IC₅₀) |
| DCAWHLGELVWCT | SEQ ID NO: 9 | 100 nM (IC₅₀) |
| | | |
| **Phage Clones (M13/gm Display)** | | All phage affinities are EC_{50S} |
| | | |
| N/A= Not individually assayed. Since they were selected for binding, EC₅₀ likely to be < 1 uM or better. | | |
| | | |
| All of the peptides listed bind IgG-Fc. | | |
| | | |
| **Focused Libraries** | | |
| | | |
| KEASCSYWLGELVWCDTLTE | SEQ ID NO: 19 | N/A |
| KEASCSYWLGELVWCSPGVE | SEQ ID NO: 20 | 734 nM |
| KEASCSYWLGELVWCSGVEG | SEQ ID NO: 21 | N/A |
| KEASCSYWLGELVWCSAGVE | SEQ ID NO: 22 | N/A |
| ESEDCSYWLGELVWCVAGVE | SEQ ID NO: 23 | N/A |
| EKEDCSYWLGELVWCVAGVE | SEQ ID NO: 24 | N/A |
| EDPDCSYWLGELVWCVAGVE | SEQ ID NO: 25 | N/A |
| EEADCSYWLGELVWCVAGVE | SEQ ID NO: 26 | N/A |
| NADD CSYWLGELVWCVAGVE | SEQ ID NO: 27 | N/A |
| SETTCSYWLGELVWCVAGVE | SEQ ID NO: 28 | N/A |
| AWKTCQWLGELVWCVAGVE | SEQ ID NO: 29 | N/A |
| DLADCSYWLGELVWCSRVEG | SEQ ID NO: 30 | 776 nM |
| KEADCAWHLGELVWCVAGVE | SEQ ID NO: 31 | 138 nM |
| KEAECSYHLGELVWCVAGVE | SEQ ID NO: 32 | N/A |
| KEARCWYWHGELVWCSDPEE | SEQ ID NO: 33 | 809 nM |
| KEASCSYHLGELVWCVAGVE | SEQ ID NO: 34 | 416 nM |
| KEASCSWHLGELVWCVAGVE | SEQ ID NO: 35 | 225 nM |
| KEASCSYWLGELVWCTEGVE | SEQ ID NO: 36 | 818nM |
| KEASCSYWLGELVWCDDGVE | SEQ ID NO: 37 | N/A |
| KEASCSYWLGELVWCSEGVE | SEQ ID NO: 38 | N/A |
| KEASCSYWLGELVWCSPGVE | SEQ ID-NO: 39 | N/A |
| KEASCSYWLGEVWKCKSGVE | SEQ ID NO: 40 | N/A |
| KEASCSYWLGELVWCDNGVE | SEQ ID NO:41 | N/A |
| KEASCSYWLGELVWCDTFDE | SEQ ID NO: 42 | 301 nM |
| KEASCSYWLGELVWCDGLDE | SEQ ID NO: 43 | 326 nM |
| KEASCSYWLGELVWCVGLDE | SEQ ID NO: 44 | 278 nM |
| KEASCSYWLGELVWCEDTLE | SEQ ID NO: 45 | N/A |
| KEASCSYWLGELVWCEDTME | SEQ ID NO: 46 | N/A |
| KEASCSYWLGELVWCEDMME | SEQ ID NO: 47 | N/A |
| WVEDCSWHMGELVWCDGGEF | SEQ ID NO: 48 | 139 nM |
| KEASCSYWLGELVWCDWMNG | SEQ ID NO: 49 | N/A |
| KEASCSYWLGELVWCDDTPV | SEQ ID NO: 50 | N/A |
| KEASCSYWLGELVWCDDYGE | SEQ ID NO: 51 | N/A |
| KEASCSYWLGELVWCSDLWE | SEQ ID NO: 52 | N/A |
| WRGGCSWHMGELVWCEHDME | SEQ ID NO: 53 | N/A |
| AVSKCSFHMGELVWCSDVMN | SEQ ID NO: 54 | N/A |
| NQVSCSYSRGELVWCSKQSQ | SEQ ID NO: 55 | N/A |
| GRMECAWHQGELVWCTPTLE | SEQ ID NO: 56 | N/A |
| GTMECSWHQGELVWCTPTLA | SEQ ID NO: 57 | N/A |
| EMRDCSWHLGELVWCAHMEG | SEQ ID NO: 58 | N/A |
| GSWECAYHLGELVWCETGSG | SEQ ID NO: 59 | N/A |
| VAEPCAYHLGELVWCEVLKG | SEQ ID NO: 60 | N/A |
| KEAMCSYWLGELVWCESDMP | SEQ ID NO: 61 | N/A |
| | | |
| **Designed Clones** | | |
| | | |
| DLADCSWHLGELVWCSRVEG | SEQ ID NO: 62 | 9 nM |
| DLADCSWHLGELVWCVGLDE | SEQ ID NO: 63 | 28 nM |
| WVEDCSWHLGELVWCVGLDF | SEQ ID NO: 64 | 31 nM |
| | | |
| **Secondary Optimization** | | |
| | | |
| KVADCAWHMGELVWCTEVEG | SEQ ID NO: 65 | 23 nM |
| GEEDCSYHLGELVMCTELDD | SEQ ID NO: 66 | 69 nM |
| GVADCAWHLGELVWCTERED | SEQ ID NO: 67 | N/A |
| GEEDCAWHLGELVWCSGGDF | SEQ ID NO: 68 | 100 nM |
| WEADCAWHLGELVWGTKVEE | SEQ ID NO: 69 | 7 nM |
| GEADCSYHLGELVWCNDFEE | SEQ ID NO: 70 | 156 nM |
| WVDCAYHLGELVWCSTFEE | SEQ ID NO: 71 | 9 nM |
| WVEDCAWHMGELVWCTKVDE | SEQ ID NO: 72 | 70 nM |
| READCAWHLGELVWCSERDL | SEQ ID NO: 73 | 47 nM |
| EEASCAYHLGELVWCDAFDV | SEQ ID NO: 74 | 77 nM |
| RVASCAWHLGELVWCDGLDG | SEQ ID NO: 75 | N/A |
| GEADCAWHLGELVWCTKVEE | SEQ ID NO: 76 | 38 nM |
| GEASCAYHLGELVWCDEGEG | SEQ ID NO: 77 | 386 nM |
| RVEDCAYHLGELVWCTEGDE | SEQ ID NO: 78 | 63 nM |
| | | |
| EEPDCSWHLGELVMCTPMEV | SEQ ID NO: 79 | 14 nM |
| KEADGAWHMGELVWCSEMEG | SEQ ID NO: 80 | 66 nM |
| EQADCAWHLGELVWCTPMVF | SEQ ID NO: 81 | 8 nM |
| EEPDCSWHLGELVWCTPIEV | SEQ ID NO: 82 | 15 nM |
| GEPDCAWHLGELVWCTPMVF | SEQ ID NO: 83 | 7 nM |
| GEQDCSYHMGELVWCTTVDG | SEQ ID NO: 84 | 210 nM |
| GVRNCAYHLGELVWCTPMEF | SEQ ID NO: 85 | 10 nM |
| RVADCAWHMGELVWCSELEV | SEQ ID NO: 86 | 44 nM |
| GEADCAWHLGELVWCTPMDG | SEQ ID NO: 87 | N/A |
| GEQDCSWHLGELVWCTPMEV | SEQ ID NO: 88 | N/A |
| GMRDCSYHLGELVWCSDMEL | SEQ ID NO: 89 | N/A |
| EVADCSWHLGELVWCTEGEF | SEQ ID NO: 90 | 54 nM |
| GEEDCAWHLGELVWCTDVED | SEQ ID NO: 91 | 52 nM |
| EVEDCAYHLGELVWCSDLEG | SEQ ID NO: 92 | 82 nM |
| WEEDCAWHLGELVWCAEFDE | SEQ ID NO: 93 | 44 nM |
| KEASCAWHLGELVWCSEVEE | SEQ ID NO: 94 | 130 nM |
| | | |
| **ALA Scan on Phage** | | |
| | | |
| AEADCAWHLGELVWCTKVEE | SEQ ID NO: 95 | 20 nM |
| WAADCAWHLGELVWCTKVEE | SEQ ID NO: 96 | 34 nM |
| WEPDCAWHLGELVWCTKVEE | SEQ ID NO: 97 | 36 nM |
| WEAACAWHLGELVWCTKVEE | SEQ ID NO: 98 | 55 nM |
| WEAACSWHLGELVWCTKVEE | SEQ ID NO: 99 | 10 nM |
| WEADCAAHLGELVWCTKVEE | SEQ ID NO: 100 | 798 nM |
| WEADCAWALGELVWCTKVEE | SEQ ID NO: 101 | 139 nM |
| WEADCAWHAGELVWCTKVEE | SEQ ID NO: 102 | 56 nM |
| WEADCAWHLAELVWCTKVEE | SEQ ID NO: 103 | 12 nM |
| WEADCAWHLGALVWCTKVEE | SEQ ID NO: 104 | 11 nM |
| WEADCAWHLGEAVWCTKVEE | SEQ ID NO: 105 | 1890 nM |
| WEADCAWHLGELAWCTKVEE | SEQ ID NO: 106 | 4670 nM |
| WEADCAWHLGELVACTKVEE | SEQ ID NO: 107 | 3380 nM |
| WEADCAWHLGELVWCAKVEE | SEQ ID NO: 108 | 101 nM |
| WEADCAWHLGELVWCTAVEE | SEQ ID NO: 109 | 10 nM |
| WEADCAWHLGELVWCTKAEE | SEQ ID NO: 110 | 8 nM |
| WEADCAWHLGELVWCTKVAE | SEQ ID NO: 111 | 4 nM |

### EXAMPLE 2

### Construction of Anti-VEGF Fabs Tagged with IgG-Fc Peptide Ligands

IgG-Fc peptide ligands may be combined with a bioactive compound to form a hybrid molecule that comprises a peptide ligand domain and an active domain. In this Example, IgG-Fc peptide ligands are combined with a Fab fragment that recognizes human VEGF. A neutralizing antibody to human VEGF has been previously identified from murine hybridoma, humanized, and optimized by phage display. *See* Muller et al. (1998), Structure 6:1153-1167; Chen et al. (1999), J. Mol. Biol 293:865-881; and International Patent Publication No. WO 98/45331. Two humanized Fab forms of this antibody were chosen to test whether binding affinity to an irrelevant IgG could be added to the Fabs without disrupting their antigen-binding affinity. An IgG-Fc peptide ligand, DCAWHLGELVWCT (SEQ ID NO: 9), identified and optimized by the peptide-phage display method described in Example I was used, along with a short peptide linker (Gly-Gly-Gly) to provide flexibility between the peptide and the Fab. The light chain of the Fab was chosen for fusions because in the case of this antibody, the light chain is known to have little contribution to antigen binding (Muller *et al.,* 1998, *supra).* In principle the peptide ligand domain could function to introduce IgG-binding whether introduced at the N-terminus; C-terminus, or inserted within the original Fab sequence. Described here are N-terminal fusions. DCAWHLGELVWCTGGG-(light chain) (SEQ ID NO: 112) as well as C-terminal fusions (light chain)-GGGWEADCAWHLGELVWCT (SEQ ID NO: 113).

An oligodeoxynucleotide, HL-569, was designed and synthesized for mutation of anti-VEGF plasmids to create fusions of the IgG-Fc peptide ligand at the N-terminus of the antibody light chain. The sequence of HL-569 (with added peptide sequence underlined) is: 5'-ACA AAC GCG TAC GCT GAC TGC GCT TGG CAC CTG GGC GAG CTG GTC TGG TGC ACC GGA GGA GGA GAT ATC CAG TTG ACC-3' (SEQ ID NO: 114). The GAC codon follows the STII secretion-signal sequence at the N-terminus of the light chain, and the GAT codon corresponds to the first residue of the mature (wild-type) light chain.

Another oligodeoxynucleotide, HL-570, was designed and synthesized for construction of peptide ligand fusions to the C-terminus of the antibody light chain. The sequence of HL-570 (with added peptide sequence underlined) is: 5'-AAC AGG GGA GAG TGT GGA GGA GAGA TGG GAA GCA GAC TGC GCT TGG CAC CTG GGC GAG CTG GTC TGG TGC ACC TAA GCT GAT CCT CTA C-3' (SEQ ID NO: 115). The TGT codon preceding the underscored GGA codon corresponds to residue Cys-214 of the light chain, and the TAA "stop codon" marks the end of the translated peptide sequence. Phagemids pY0192 and pY0317 (described Muller *et al.,* 1998, *supra;* Chen *et al.,* 1999; and International Patent Publication No. WO 98/45331, encoding low-affinity and high-affmity forms of a humanized anti-VEGF antibody, respectively, were mutated with each of the two IgG-peptide oligos to yield constructs pY0192-569, pY0192-570, pY0317-569, and pY0317-570.

### EXAMPLE 3

### Phage-ELISA Analysis of Hybrid Molecules Comprising Peptide-Ligand Tagged Anti-VEGF Fabs

A phage-ELISA competitive binding assay (Lowman (1998), *Methods Mol. Biol.* **87**:249-264) was used to compare the apparent binding affinities of anti-VEGF antibody variants tagged with an IgG-Fc peptide ligand at their N-terminus or C-terminus and displayed monovalently on bacteriophage M13 particles as fusions to the C-terminal domain of the gene III protein.

An irrelevant humanized IgG, 4D5-IgG, also known as Herceptin®, was coated onto Nunc Maxisorp immunosorbant plates at 2 microg/mL in phosphate buffered saline solution (PBS). Phagemid particles from overnight cultures of XL-1 Blue *E*. *coli* (Stratagene) were diluted in PBS containing 0.5% bovine serum albumin and 0.05% Tween-20. The phagemid particles were mixed with serial dilutions of Herceptin® in solution, equilibrated for 20 min in a non-adsorbent plate (Nunc F96), then transferred to the Herceptin®-coated Maxisorp plate for detection of unbound phage. After 20 min, the plate was washed with PBS/Tween, and developed with an anti-phage monoclonal antibody-HRP conjugate (Pharmacia) and OPD substrate (Sigma). Displacement curves (Fig. 1) showed IC₅₀ values of about 100-300 nM for each of the constructs, pY0192-569, pY0192-570, pY0317-569, and pY0317-570.

### EXAMPLE 4

### BIAcore^{™} Analysis of IgG Binding to Anti-VEGF Fab Tagged with an IgG-Fc Peptide Ligand

A surface plasmon resonance instrument (BIAcore, Inc., Piscataway, N.J.) was used to measure binding of an irrelevant IgG, 4D5-IgG, also known as Herceptin®, to Fab that previously had been bound to an immobilized VEGF biosensor chip.

Fab variants encoded by pY0317 and pY0317-570 (control anti-VEGF high affinity, humanized Fab, and anti-VEGF high affinity, humanized Fab tagged with an IgG-Fc peptide ligand domain, respectively; *see* Example 2, *supra,* and WO 98/45331) were expressed in *E*. *coli* and purified by protein-G (Pharmacia) affinity chromatography. Recombinant human VEGF was immobilized onto BIAcore^{™} CM-5 biosensor chips (BIAcore, Inc.) as described (Muller *et al.,* 1998, *supra*). After VEGF immobilization, the chip was blocked with ethanolamine, and the peptide-ligand tagged Y0317-570 Fab, or Y0317 control, was injected in PBS buffer containing 0.05% Tween-20 and 0.0 1 % sodium azide. Following Fab injection, Herceptin® was injected, and the dissociation off-rate (k_{off}) following injection was observed.

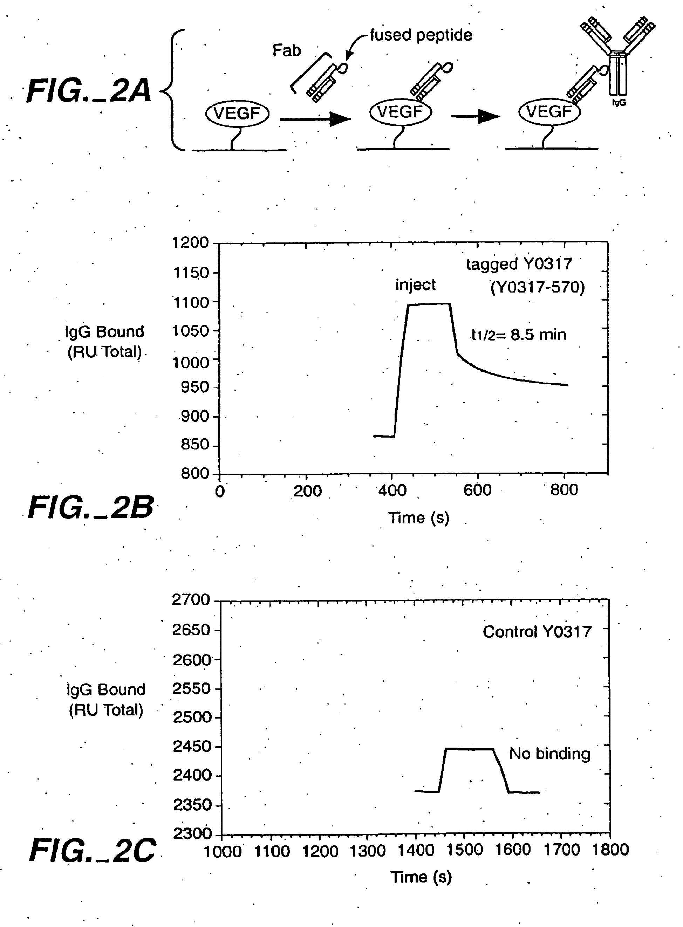

The results (Fig. 2) show that Herceptin® bound to the tagged but not to the control Y0317 Fab. Using as 1:1 Langmuir binding model (Karlsson et al. (1991), J. Immunol. Methods 145:229-240 (1991)), a k_{off} of 2.8 x 10-3, sec⁻¹, and a corresponding dissociation half-life (t_{1/2}) of 8.5 min were calculated for Y0317-570. Limitations of material prevented reliable on-rate determinations. However, the observed k_{off} suggests an equilibrium binding affinity, K_{d}, of 30 nM to 300 nM (assuming kₒₙ of 10⁴-10⁵ M⁻¹ sec-¹), consistent with peptide binding and phage-ELISA results (above). Importantly, the BIAcore^{™} results (Fig. 2) also show that the tagged Fab can simultaneously binding both antigen (immobilized VEGF) and an irrelevant IgG.

### EXAMPLE 5

### IgG-Fc Peptide Ligand Tagged Anti-VEGF Fabs. Have Prolonged Elimination Half Times

The blood clearance rates and tissue distribution of the IgG-Fc peptide ligand-tagged anti-VEGF Fab (Fab-Y0317-570) are compared to those of the untagged control anti-VEGF Fab Y0317. Determinations of the elimination half time and volume of distribution are made in New Zealand White Rabbits of 2.8 to 3 kg weight. The amount of test article present in the plasma samples is determined using any method known in the art, such as, e.g., ELISA, or RIA.

Pharmacokinetic analysis is performed using the test article plasma concentrations. Group mean plasma data for each test article conforms to a multi-exponential profile when plotted against the time post-dosing. The data are fit by a standard two-compartment model with bolus input and first-order rate constants for distribution and elimination phases. The general equation for the best fit of the data for i.v. administration is: *c(t)* = *Ae-^{αt}* + *Be*^{-βt}, where c(t)- is the plasma concentration at time t, A and B are intercepts on the Y-axis, and α and β are the apparent first-order rate constants for the distribution and elimination phases, respectively. The α-phase is the initial phase of the clearance and reflects distribution of the protein into all extracellular fluid of the animal, whereas the second or β-phase portion of the decay curve represents true plasma clearance. Methods for fitting such equations are well known in the art. For example, *A* = D/V(α-k21)/(α-β), *B* = D/V (β-k21)/(α-β), and α and β (for α > β) are roots of the quadratic equation: r² + (k12 + k21 + k10)r + k21k10 = 0 using estimated parameters of V = volume of distribution, k10 = elimination rate, k12 = transfer rate from compartment 1 to compartment 2 and k21 = transfer rate from compartment 2 to compartment 1, and D = the administered dose.

On the morning of the study six New Zealand White rabbits (body weight 2.8-3.0 kg) were placed in restrainers. Catheters were installed in an ear artery for blood sample collection and in a contralateral ear vein for dosing.

Rabbits were divided into two groups (n=3/group). Group 1 animals received and IV bolus of control anti-VEGF Fab-Y0317. Rabbits in Group 2 received Fab-Y0317-570. A summary of group assignment and dosing information is presented in the table below.

| Group | Weight (kg) | Dose Group | Nominal Dose (mg/kg) | Dose Conc. (mg/mL) | Dose Vol. (mL) |
|---|---|---|---|---|---|
| 1 | 2.9 | Control-Fab-Y0317 | 1 | 3 | 0.97 |
| 1 | 3.0 | Control-Fab-Y0317 | 1 | 3 | 1.00 |
| 1 | 2.9 | Control-Fab-Y0317 | 1 | 3 | 0.97 |
| | | | | | |
| 2 | 2.8 | Fab-Y0317-5701 | | 3 | 0.93 |
| 2 | 3.0 | Fab-Y0317-5701 | | 3 | 1.00 |
| 2 | 2.9 | Fab-Y0317-5701 | | 3 | 0.97 |

Serial blood samples (0.5 mL) were collected just prior to dosing and at 10, 20 40 min, 1, 2, 3, 4, 6, 8, 24 and 48 hr after dose administration. Blood was collected in serum separator tubes, allowed to clot (∼30 min) at room temperature, and centrifuged. Serum was harvested and immediately stored at -70C until analyzed.

ELISA plates were coated with 0.5 microg/ml VEGF in 50 mM carbonate buffer, pH 9.6, at 4°C overnight and blocked with 0.5% bovine serum albumin, 10 ppm Proclin 300 (Supelco, Bellefonte, PA) in PBS (8 mM Na₂HPO₄, 1.5 mM KH₂PO₄, 2.7 mM KCl and 137 mM NaCl, pH 7.2) at room temperature for 1 hour. Standards (0.41-100 ng/ml) and twofold serial dilutions of samples (minimum dilation 1: 100) in PBS containing 0.5% bovine serum albumin, 0.05% polysorbate 20, 0.25% CHAPS, 0.2% bovine gamma globulins (Sigma, St. Louis, MO) and 5 mM EDTA were incubated on the plates for 2 hours. Bound antibody was detected using peroxidase labeled goat F(ab')2 anti-human IgG f(ab')2 (Jackson ImmunoResearch, West Grove, PA), followed by 3,3',5,5'-tetramethyl benzidine (Kirkegaard & Perry Laboratories) as the substrate. Plates were washed between steps. Absorbance was read at 450 nm on a Titerek stacker reader (ICN, Costa Mesa, CA). The standard curve was fitted using a four-parameter regression curve-fitting program (Kaleidagraph, Synergy Software, Reading, PA). Data points which fell in the range of the standard curve were used for calculating the Fab concentrations in samples.

Data analysis: Graphs of concentration versus time profiles were made using KaleidaGraph (KaleidaGraph^{™} V. 3.09 Copyright 1986-1997. Synergy Software. Reading, PA.). Values reported as less than reportable (LTR) were not included in the PK analysis and are not represented graphically. Pharmacokinetic parameters were determined by compartmental analysis using WinNonlin software (WinNonlin^{®} Professional V. 3.1 WinNonlin^{™} Copyright 1998-1999. Pharsight Corporation. Mountain View, CA. ). Pharmacokinetic parameters were computed as described elsewhere (Ritschel WA and Keams GL. Handbook of basic pharmacokinetics including clinical applications, 5th edition. American Pharmaceutical Assoc., Washington, DC. Copyright 1999).

The results are reported in Figure 3. A two-compartment model with bolus input and first-order output (WinNonlin) was used to fit observed serum concentration vs. time data. Calculated pharmacokinetic parameters ware presented in the table below.

| | Pharmacokinetic Parameter Summary (IV bolus; 1 mg/kg) | |
|---|---|---|
| Parameter | Group 1 | Group 2 |
| | Control Fab-Y0317 | Fab-Y0317-570 |
| AUC (h*µg/mL) | 13.6 ± 1.2 | 215 ± 56 |
| Cmax (µg/mL) | 15.6±0.6 | 13±0.7 |
| CL (mL/h/kg) | 74.2±6.7 | 4.8±1.1 |
| K10 half-life (hr) | 0.6±0.02 | 11.3±3.6 |
| alpha half-life (hr) | 0.39±0.03 | 1.15±0.31 |
| beta half-life (hr) | 1.93±0.27 | 37.6±19 |
| V 1 (mL/kg) | 64.1±2.37 | 75.2±4.23 |
| Vss (mL/kg) | 112±7.7 | 225±54 |

The initial volume of distribution (V1) for both agents was approximately equal to serum volume. The estimated steady state volume of distribution (Vss) for Fab-Y0317-570 (225 mL/kg) was approximately 2 fold higher than estimated for the control Fab (112 mL/kg) suggesting a significant amount of binding to endogenous IgG. Control Fab-Y0317 was eliminated approximately 15-fold faster from the serum (clearance = 74 mL/h/kg) compared to Fab-Y0317-570 (4.8 mL/h/d). The overall exposure (AUC) of Fab-Y0317-570 was ∼16 times higher than for Fab-Y0317. Fab-Y0317 was undetectable in the serum 24 h after dosing but serum concentrations of Fab-Y0317-570 were still above 1 µg/mL 48 h after dosing. Both the distribution (alpha) half-life (1.15 h) and the elimination (beta) half-life (37.6 h) were significantly longer than the control Fab.

These results suggest that addition of a 13 amino acid that binds to endogenous IgG to Fab-Y0317 can significantly slow Fab clearance, increase half-life and enhance overall exposure.

### EXAMPLE 6

### Serum Albumin Peptide Ligands

Phage Libraries and Selection Conditions-Phage-displayed peptide libraries were selected against rabbit, rat and human albumin. Phage libraries expressing random peptide sequences fused to gene 8 (Lowman et al., Biochem. 37, 8870 (1998)) were pooled into 5 groups: Pool A contained CX₂GPX₄C, X₄CX₂GPX₄CX₄ and XᵢCXⱼCXₖ where j = 8-10; Pool B contained X₂₀ and XᵢCXⱼCXₖ where j = 4-7; Pool C contained X₈ and X₂CXⱼCX₂ where j = 4-6; Pool D contained X₂CXⱼCX₂ where j = 7-10 ; Pool E contained CX₆CX₆CCX₃CX₆C, CCX₃CX₆C, CCX₅CX₄CX₄CC, CXCX₇CX₃CX₆ where X represents any of the 20 naturally occurring L-amino acids. In each case i + j + k = 18 and) | i - k | < 2. Each of the 10 libraries has in excess of 108 clones.

The phage library pools were suspended in binding buffer (PBS, 1% ovalbumin, 0.005% Tween 20) and sorted against rabbit, rat or human albumin immobilized directly on maxisorp plates (10 µg/ml in PBS, overnight at 4°C; plates were blocked with Blocker Casein (Pierce Chemical, Rockford, IL)). After 2' hours, unbound phage were removed by repetitive washing (PBS, 0.05% Tween 20) and bound phage were eluted with 500 mM KCl, 10 mM HCl, pH 2. Eluted phage were propagated in XL1-Blue cells with VCSM13 helper phage (Stratagene, La Jolla, CA). Enrichment was monitored by titering the number of phage that bound to an albumin coated well compared to a well coated with ovalbumin or casein.

Phage ELISA-Phage clones (~10¹¹ phage) were added to plates coated with rat, rabbit or human albumin. The microtiter plate was washed with wash buffer and bound phage were detected with HRP/Anti-M13 Conjugate. The amount of HRP bound was measured using ABTS/H₂O₂ substrate and monitoring the change at 405 nm.

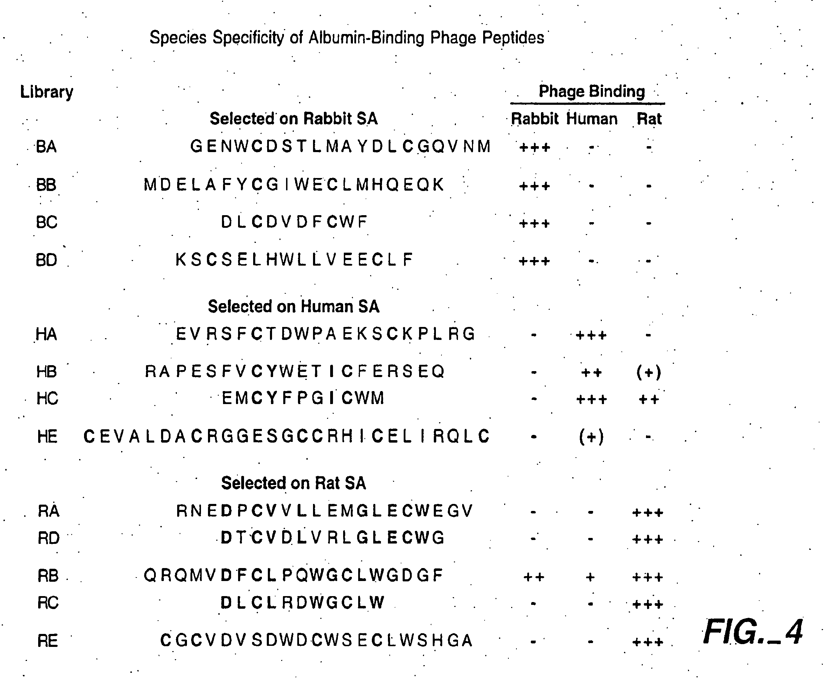

The peptide sequences displayed by phage clones selected for binding to rabbit, human or rat albumin are shown in Figure 4. Also indicated is the ability of individual phage clones to bind the 3 species of immobilized albumin. This was tested using a phage ELISA. Note that clone RB, selected for binding to rat albumin is also capable of binding human and rabbit albumin.

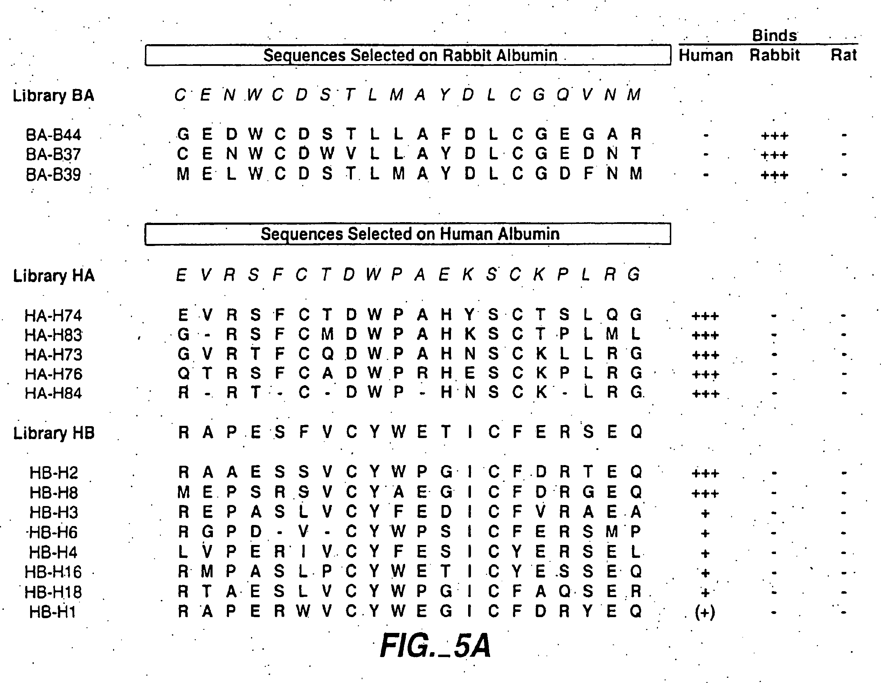

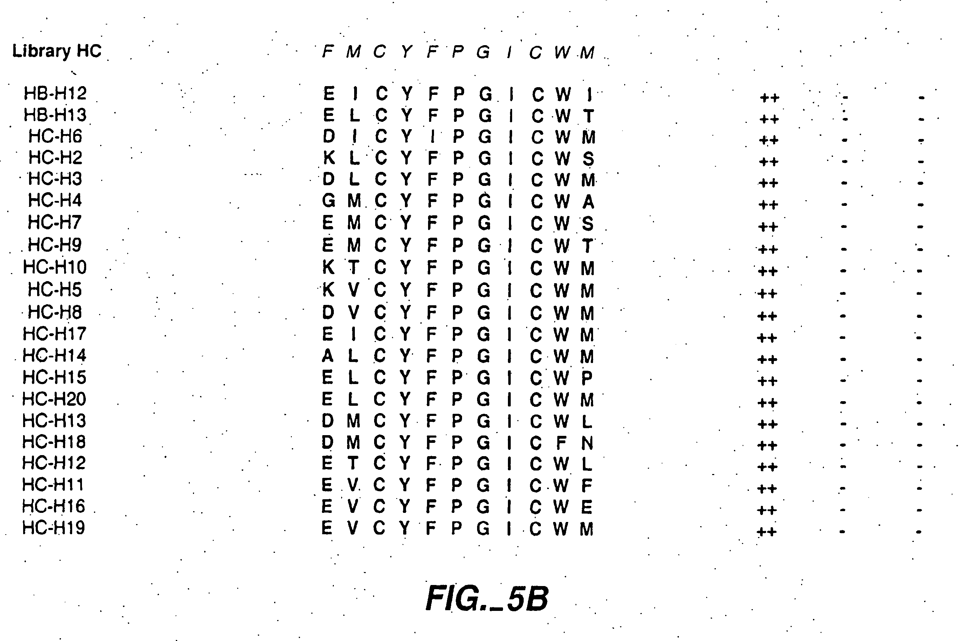

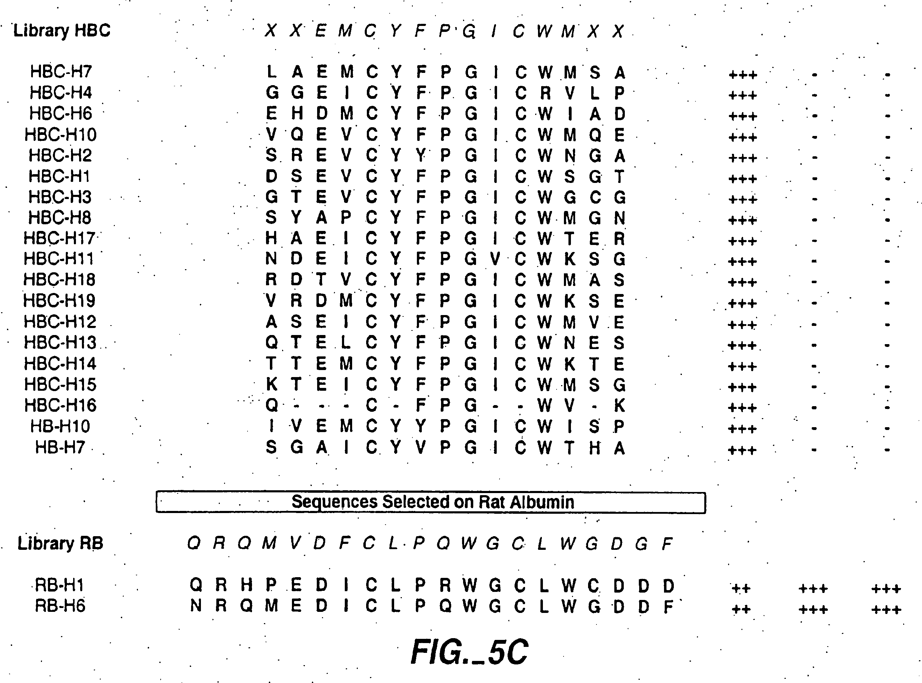

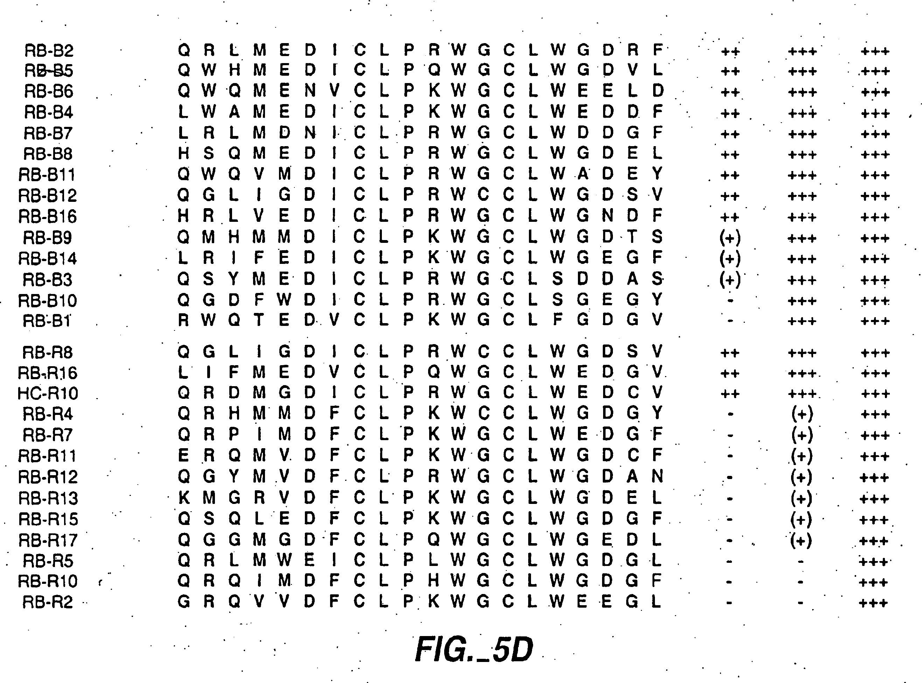

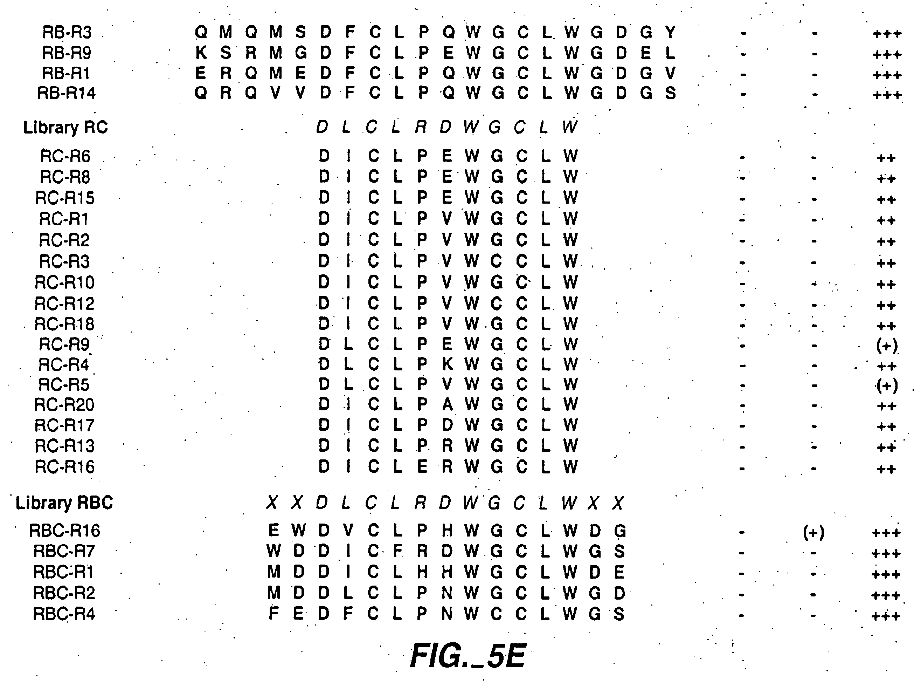

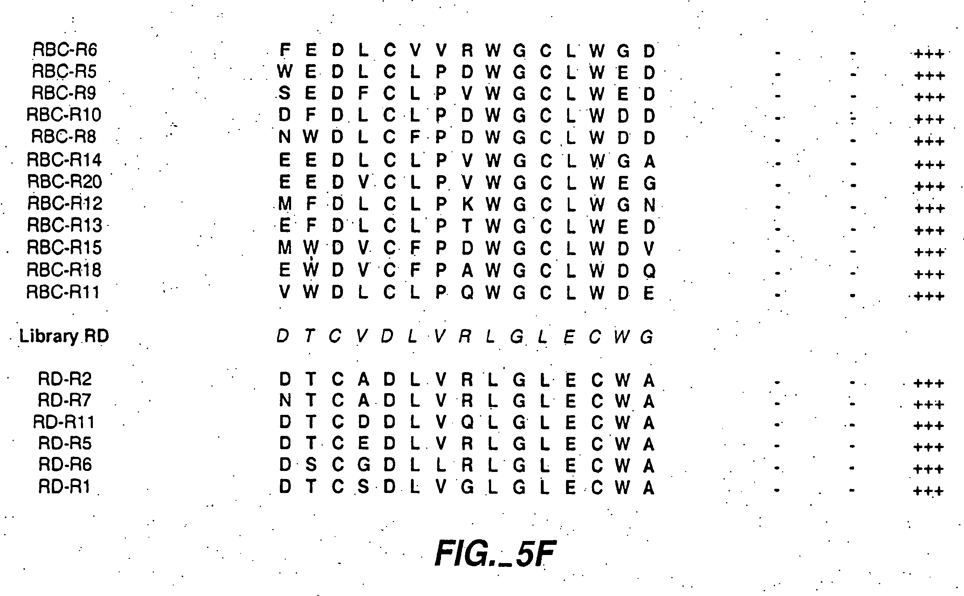

Sequence Maturation on Monovalent Phage-Partially randomized libraries were designed using oligonucleotides coding for each of the selected clones in Figure 4, but synthesized with a 70-10-10-10 mixture of bases as described (Dennis et al., Nature 404, 465 (2000)). Although the potential diversity of these libraries is the same as the initial naive libraries, each 'soft randomized' library maintains a bias towards the selected sequence in Figure 4. Each library was again selected for binding to rat, rabbit or human albumin regardless of its origin. For example, the library resulting from soft randomization of clone RB was selected against rat, rabbit or human albumin even though it was originally identified for binding to rat albumin. Sequences identified following soft randomization are shown in Figure 5 along with their species specificity as determined by phage ELISA. Most clones appear to be specific for the species of albumin for which they were selected, however, clones from the RB soft randomization library bind to all three species.

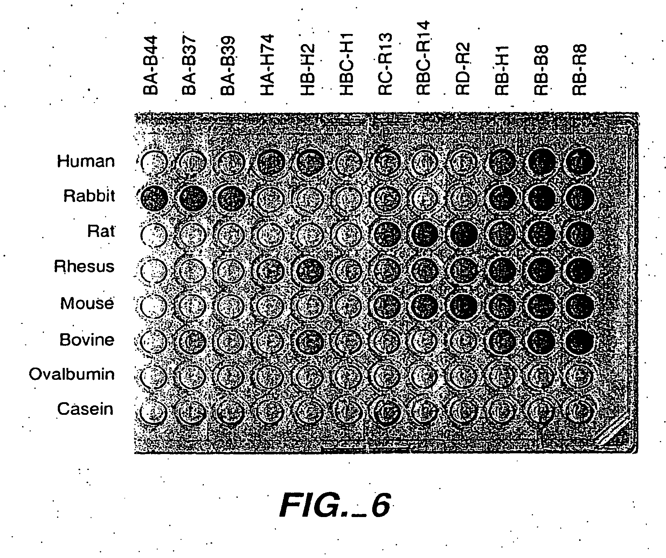

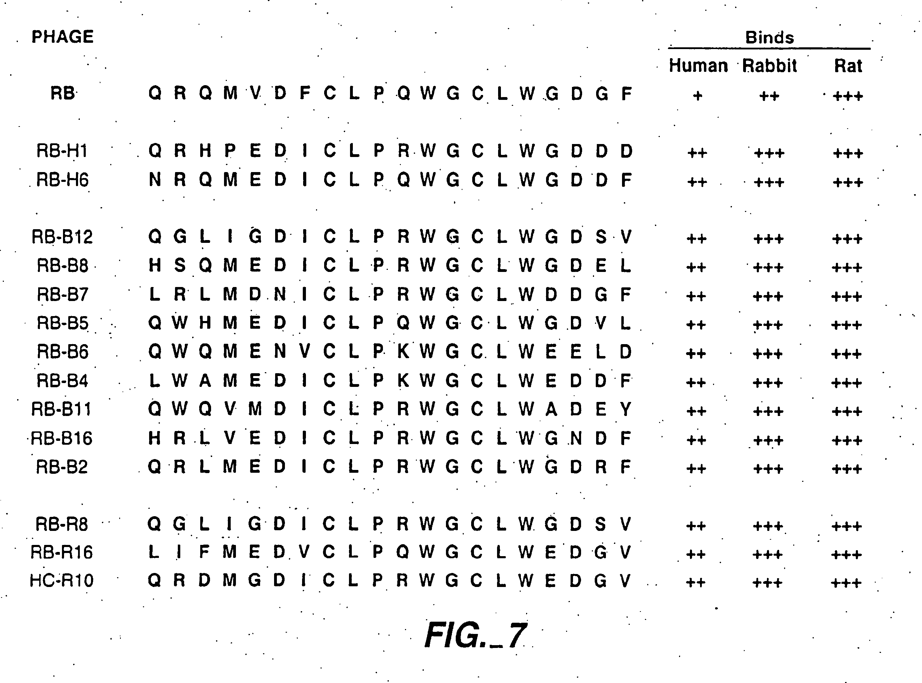

Phage clones were also tested for binding to rhesus, mouse and bovine albumin. Clones originating from the RB soft randomization library were found to bind each of these species of albumin as well and were specific for albumin based upon their lack of binding to ovalbumin and casein (Figure 6). Clones that bind to multiple species of albumin (multi-species binders) are listed in Figure 7.

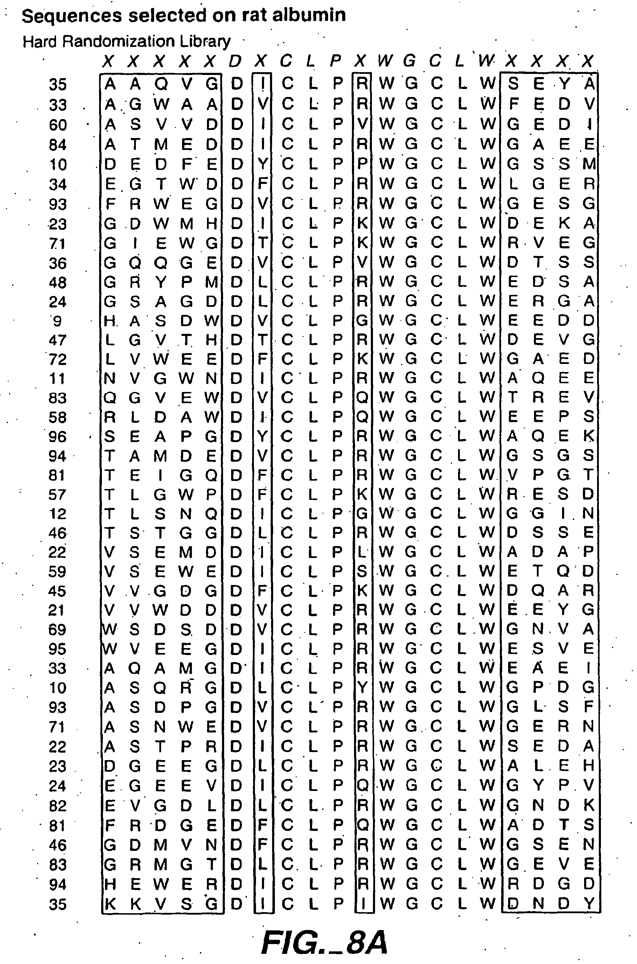

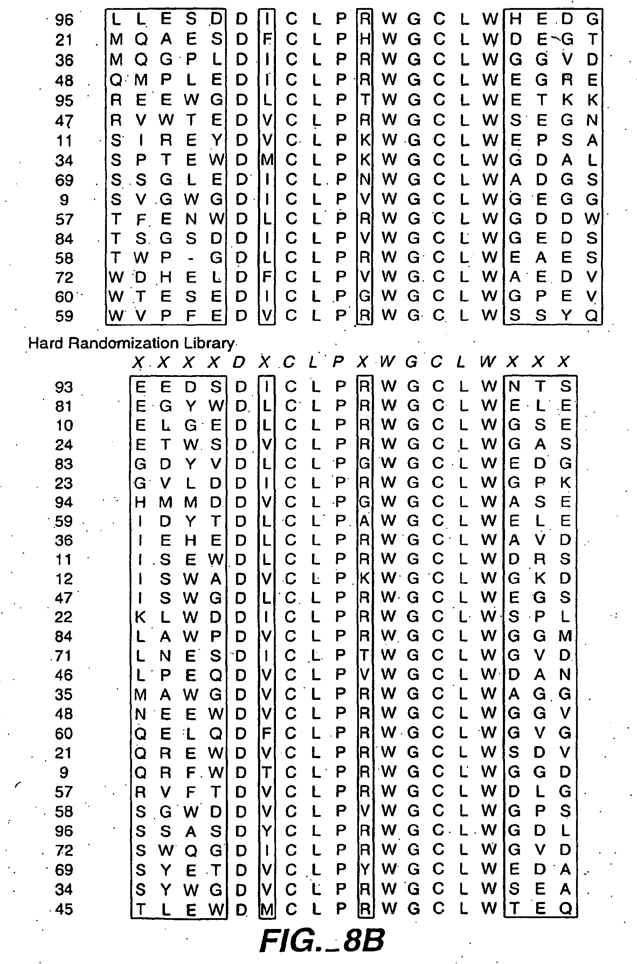

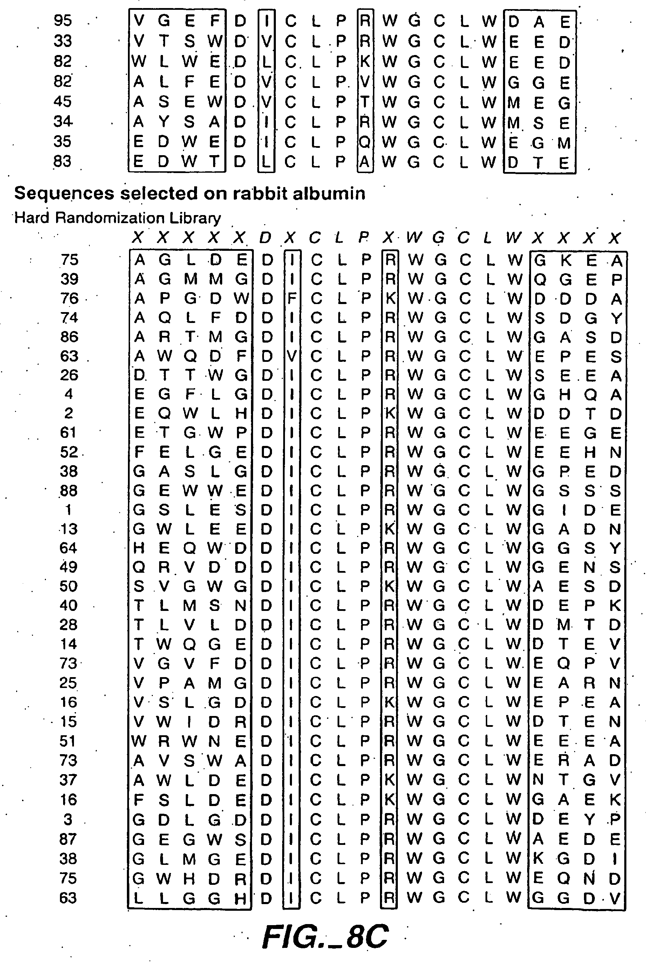

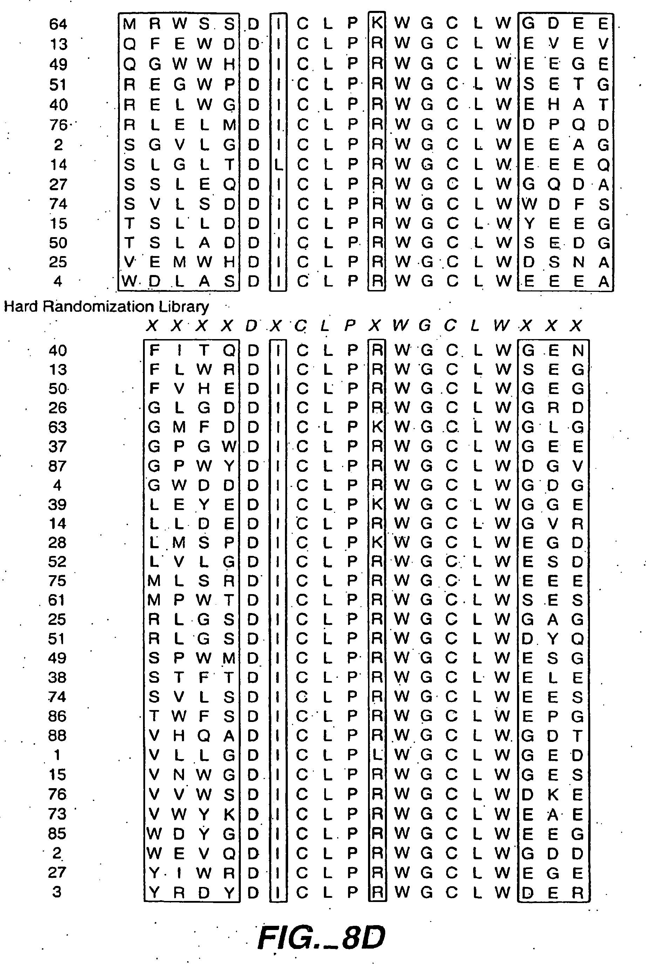

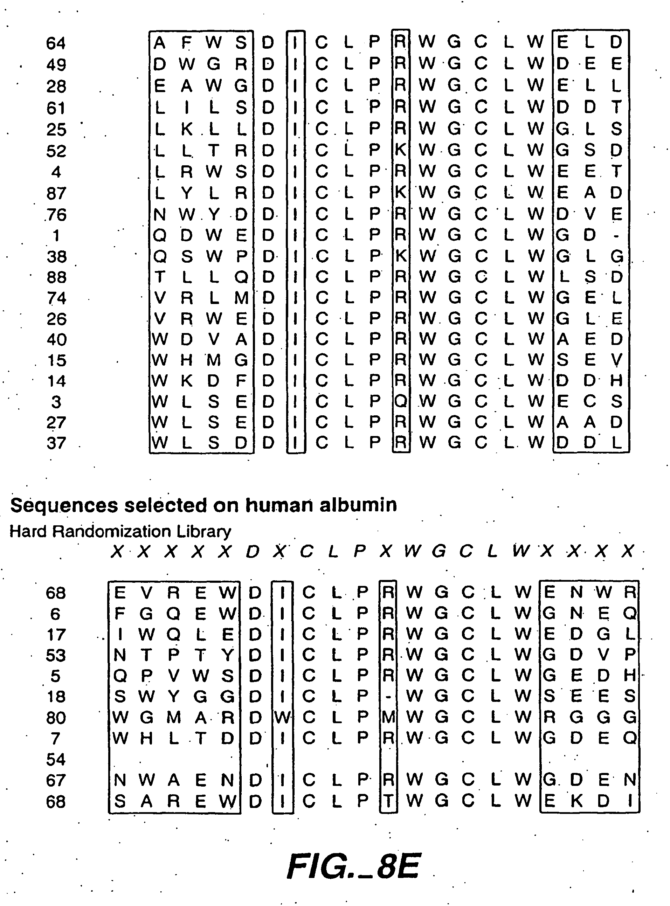

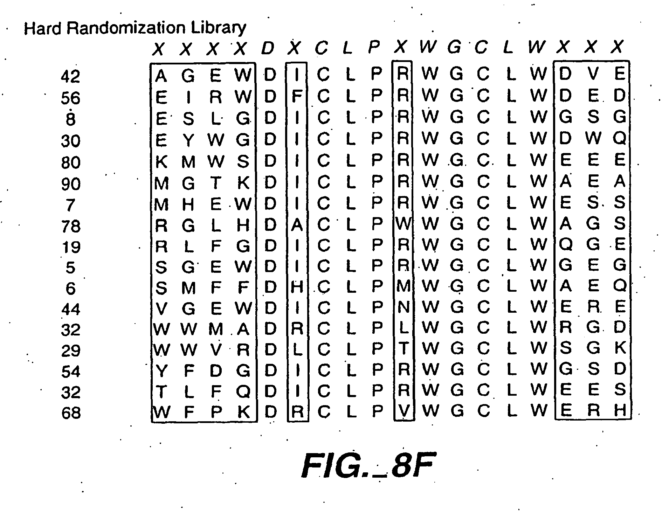

Hard randomization-Sequences from soft randomization of the RB sequence were further matured using a hard randomization strategy. A new library was designed that kept highly selected residues (underlined) constant X₅DXCLPXWGCLWX₄, while fully randomizing the remaining positions. A second library, one residue shorter at both the N and C terminus was also constructed. Sequences from these libraries selected against rat, rabbit and human albumin are shown in Figures 8A, 8B, and 8C respectively.

Peptide Synthesis-Peptides were synthesized by either manual or automated (Milligen 9050) Fmoc-based solid (phase synthesis on a 0.25 mmol scale using a PEG-polystyrene resin (Bodanszky M., (1984) Principles of Peptide Synthesis, Springer, Berlin). Side chain protecting groups were removed and the peptides were cleaved from the resin with 95% trifluoroacetic acid (TFA) and 5% triisopropylsilane. A saturated iodine solution in acetic acid was added for oxidation of disulfide bonds. Peptides were purified by reversed phase HPLC using a water/acetonitrile gradient containing 0.1% TFA. Peptides were >95% pure by analytical HPLC and its identity verified by mass spectrometry.

The carboxy terminal lysine of peptide SA08 was derivatized with NHS-LC-biotin (Pierce Chemical, Rockford, IL) and purified by HPLC as above yielding SA08b ( Ac-QGLIGDICLPRWGCLWGDSVK_{b}-n where K_{b} refers to lysine-biotin).

SA08b Binding Assay-Rabbit, rat or mouse albumin was immobilized directly on maxisorp plates at 10 µg/ml in PBS, overnight at 4°C. Plates were blocked using Blocker Casein (Pierce Chemical, Rockford, IL), for1 hr, at 25°C. Serially diluted samples were suspended in binding buffer (above) and added to the plate followed by the addition of 10 nM SA08b for 1 hr, at 25°C. The microtiter plate was washed with PBS, 0.05 % Tween 20 and the SA08b bound to albumin was detected with Streptavidin/HRP. The amount of HRP bound was measured using ABTS/H₂O₂ substrate and monitoring the change at 405 nm.

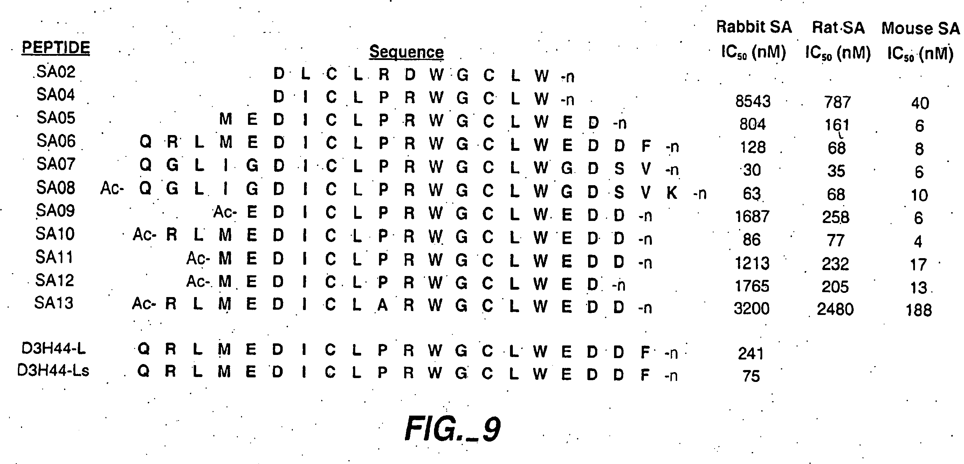

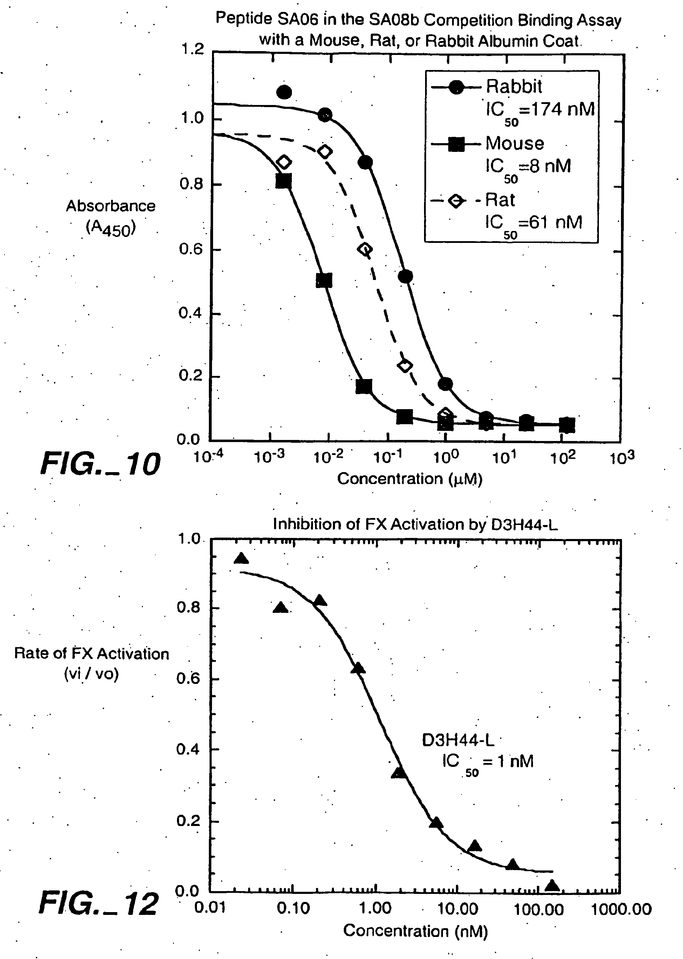

Peptides corresponding to identified phage sequences were synthesized and their affinity for rat, rabbit or mouse albumin measured using the SA08b binding assay (Figure 9 and 10).

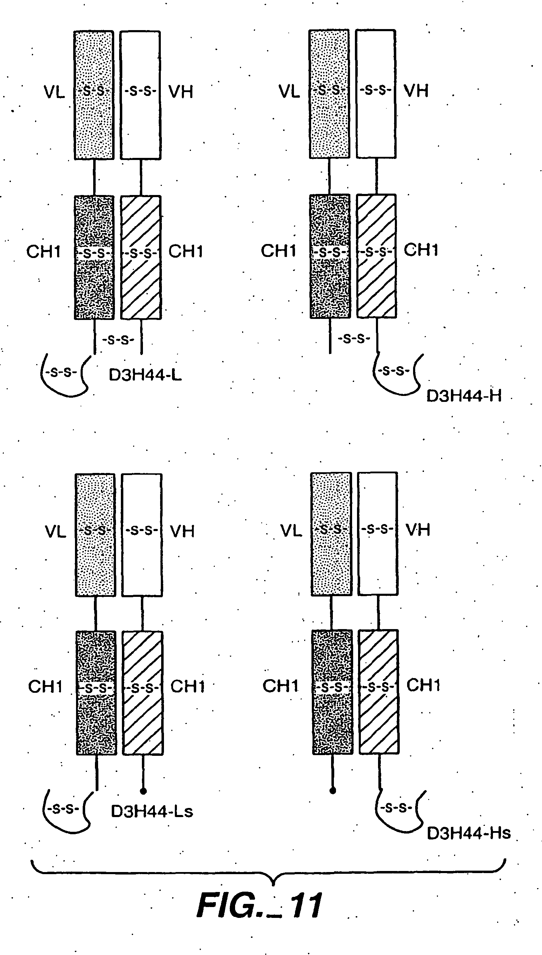

Construction, Expression and Purification of Albumin Binding Fab Fusions-In order to test whether association with albumin could increase the half-life of proteins and peptides in vivo, the sequence of SA06 was fused to a Fab fragment (D3H44) directed for binding tissue factor (TF). The SA06 sequence was added to the carboxy terminus of either the light chain (D3H44-L) or heavy chain (D3H44-Ls) of the Fab. In addition, as a precaution against folding problems, identical constructions were made but with the intra-chain disulfide replaced by alanines (D3H₄₄-Ls and D3H44-Hs, respectively) as depicted in Figure 11.

The fusions were expressed under control of the alkaline phosphatase promoter and secreted from E. coli using the stII secretion signal. Fab fusions were recovered from the periplasm by suspending cells in 1 mM EDTA, 10 mM Tris-HCl, pH8, for 1 hr at 4°C. Cell debris was removed by centrifugation and the anti-TF Fab was selectively purified using a Hi-Trap (Amersham Pharmacia Biotech, Piscataway, NJ) TF affinity column. Properly folded D3H44-L or D3H44-Ls was further purified using a rabbit albumin affinity column (rabbit albumin coupled to CNBr-activated Sepharose 4B, Amersham Pharmacia Biotech, Piscataway, NJ). Both columns were washed with PBS and eluted with 50 mM HCl. Eluted fractions were neutralized with 1 M Tris pH 8. Endotoxin was further removed following extraction with triton X114 (Aida and Pabst, J. Immunol. Methods 132, 191 (1990)).

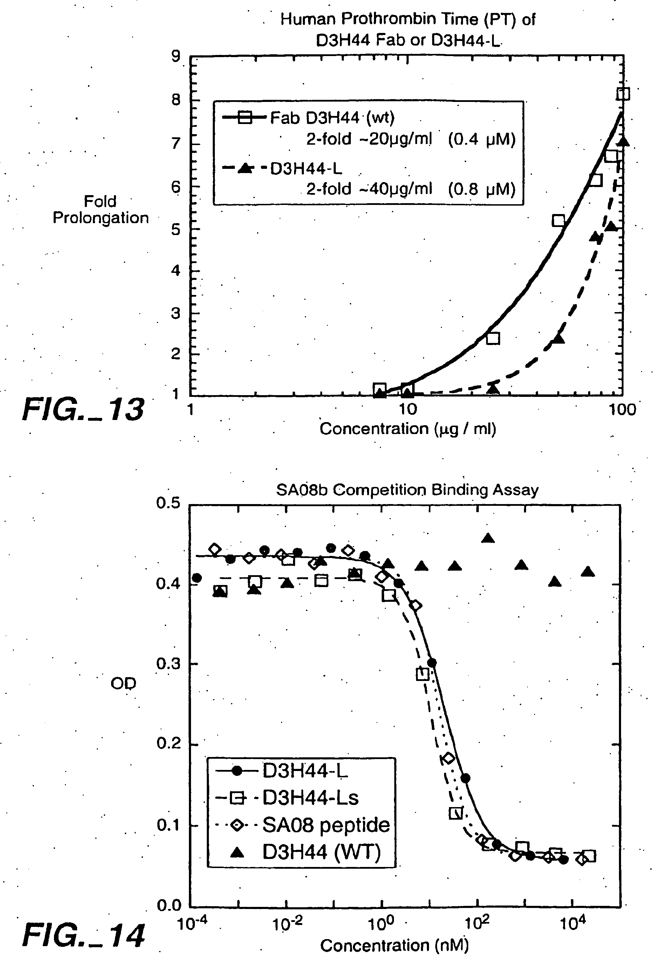

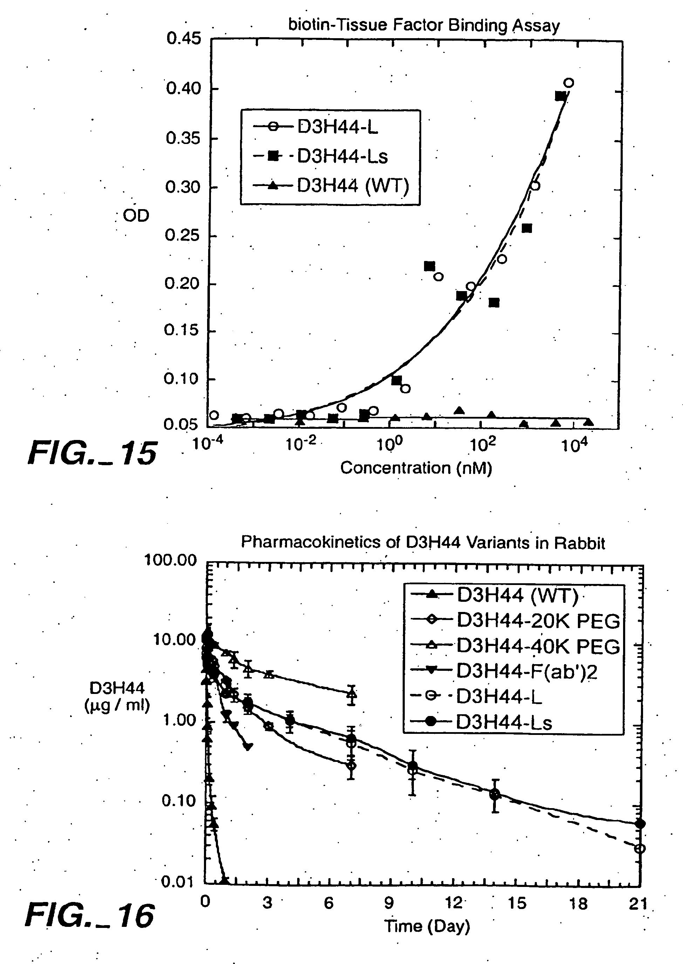

Purified D3H44 fusions retained their ability to bind TF as measured using a FX activation assay (Figure 12), and a prothrombin time assay that measures prolongation of tissue factor dependent clotting (Figure 13)(for methods see Dennis et al., Nature 404, 465 (2000)). Unlike D3H44 lacking the albumin binding sequence (WT), both D3H44-L and D3H44-Ls are able to bind to albumin as measured in the SA08b binding assay (Figure 14). Further, both D3H44 albumin-binding fusions are capable of binding TF and albumin simultaneously as judged by a biotin-TF binding assay (Figure 15). In this assay, the binding of the D3H44 fusions to immobilized albumin is detected with biotinylated TF. Wild-type D3H44 (WT) is unable to bind albumin and thus does not generate a signal upon addition of biotinylated TF.

Pharmacokinetics of D3H44 albumin-binding fusions-D3H44 variants were given as a 0.5 mg/kg bolus in rabbit. Each group consisted of 3 rabbits (5 in the F(ab')2 group). Serum samples taken at the indicated time points were serially diluted and the concentration of D3H44 determined using a TF binding ELISA.

Pharmacokinetic analysis is performed using the test article plasma concentrations. Group mean plasma data for each test article conforms to a multi-exponential profile when plotted against the time post-dosing. The data are fit by a standard two-compartment model with bolus input and first-order rate constants for distribution and elimination phases. The general equation for the best fit of the data for i.v. administration is: *c(t)* = *Ae*^{-αt} + *Be*^{-βt}, where *c*(*t*) is the plasma concentration at time *t*, A and B are intercepts on the Y-axis, and α and β are the apparent first-order rate constants for the distribution and elimination phases, respectively. The α-phase is the initial phase of the clearance and reflects distribution of the protein into all extracellular fluid of the animal, whereas the second or β-phase portion of the decay curve represents true plasma clearance. Methods for fitting such equations are well known in the art. For example, *A* = D/V(α-k21)/(α-β), *B* = D/V ^{·} (β-k21)/(α-β), and α and β (for α > β are roots of the quadratic equation: r² + (k12 + k21 + k10)r + k21k10 = 0 using estimated parameters of V = volume of distribution, k10 = elimination rate, k12 = transfer rate from compartment 11 to compartment 2 and k21 = transfer rate from compartment 2 to compartment 1, and D = the administered dose.

Data analysis: Graphs of concentration versus time profiles were made using KaleidaGraph (KaleidaGraph™ V. 3.09 Copyright 1986-1997. Synergy Software. Reading, PA.). Values reported as less than reportable (LTR) were not included in the PK analysis and are not represented graphically. Pharmacokinetic parameters were determined by compartmental analysis using WinNonlin software (WinNonlin® Professional V. 3.1 WinNonlin™ Copyright 1998-1999. Pharsight Corporation. Mountain View, CA. ). Pharmacokinetic parameters were computed as described elsewhere (Ritschel WA and Kearns GL. Handbook of basic pharmacokinetics including clinical applications, 5th edition. American Pharmaceutical Assoc., Washington, DC. Copyright 1999).

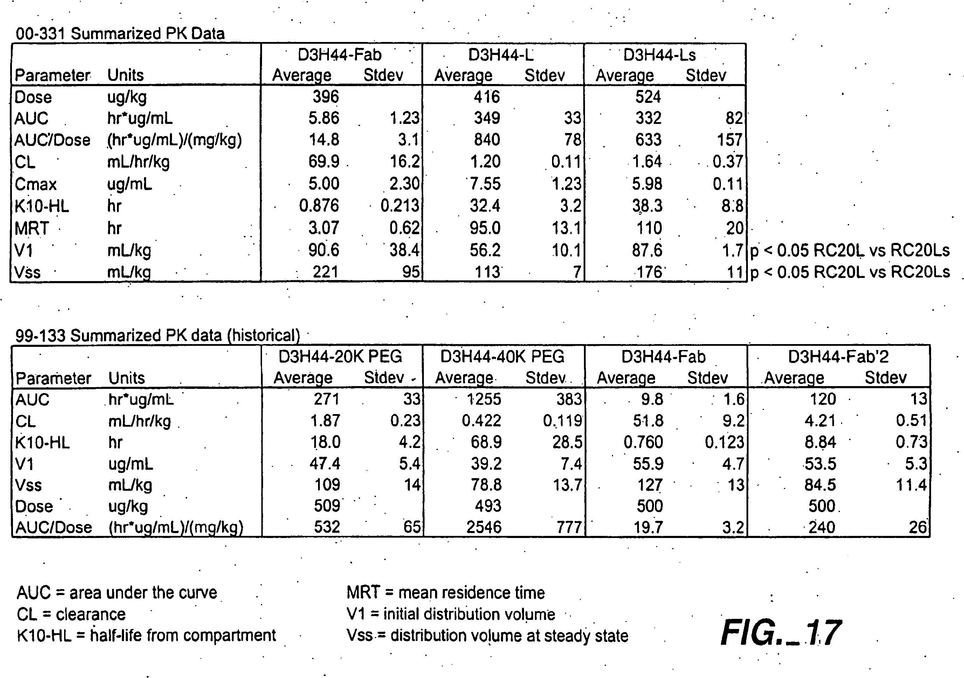

Fusion of the albumin binding peptide to D3H44 .results in a protein having improved pharmacokinetic parameters (Figure 16 and 17). D3H44-L has a 70-fold increase in half-life (K10-HL) relative to wild-type Fab and a comparable half-life to D3H44 Fabs derivatized with 20K or 40K polyethylene glycol (PEG).

## Claims

1. A peptide ligands comprising an amino acid sequence Xaaᵢ-cys-Xaaⱼ-Cys-Xₖ that binds IgG-Fc, wherein Xaa is any amino acid, Xᵢ is 0-4 amino acids, Xaaⱼ is Xaa-Xaa-Xaa-Xaa-Xaa-Xaa-Leu-Val-Trp (SEQ ID NO:9), Xₖ is 0-5 amino acids.

2. A peptide ligand according to claim 1 wherein Xaaⱼ is Xaa-Xaa-Xaa-Xaa-Gly-Glu-Leu-Val-Trp (SEQ ID NO:10).

3. A peptide ligand according to claim 2 wherein Xaaⱼ is Xaa₁-Xaa₂-Xaa₃-Xaa₄-Gly-Glu-Leu-Val-Trp-Cys and
Xaa₁ is Ala, Ser, or Thr,
Xaa₂ is Trp or Tyr,
Xaa₃ is His or Trp,
Xaa₄ is Leu or Met.

4. The peptide ligand of claim 1 or claim 2 comprising the amino acid sequence Xaa-Xaa-Xaa-Xaa-Cys-Xaa-Xaa-Xaa-Xaa-Xaa-Xaa-Leu-Val-Trp-Cys-Xaa-Xaa-Xaa-Xaa-Xaa (SEQ ID NO: 11), wherein Xaa is any amino acid.

5. A peptide according to claim 4 comprising the amino acid sequence Xaa-Xaa-Xaa-Xaa-Cys-Xaa-Xaa-Xaa-Xaa-Gly-Glu-Leu-Val-Trp-Cys-Xaa-xaa-xaa-Xaa-Xaa (SEQ ID NO:12).

6. A peptide according to claim 5 comprising the amino acid sequence Xaa₁-Xaa₂-Xaa₃-Xaa₄-Cys-Xaa₅-Xaa₆-Xaa₇-Xaa₈-Gly-Glu-Leu-Val-Trp-Cys-Xaa₉-Xaa₁₀-Xaa₁₁-Xaa₁₂-Xaa₁₃ (SEQ ID NO:13), wherein
Xaa₅ is Ala, Ser, or Thr,
Xaa₆ is Trp or Tyr,
Xaa₇ is His or Trp,
Xaa₈ is Leu or Met, and
Xaa₁₋₄ and ₉₋₁₃ are each an amino acid.

7. A peptide according to claim 6 wherein Xaa₄ is Ser, Arg, or Asp and Xaa₉ is Glu, Ser, Thr or Val (SEQ ID NO: 14).

8. A peptide according to any one of the preceding claims comprising the amino acid sequence DCAWHLGELVWCT (SEQ ID NO: 8).

9. A peptide ligand according to claim 1 comprising an amino acid sequence selected from the group consisting of;
| **SEQ ID NO:** | **AMINO ACID SEQUENCE** |
|---|---|
| 15. | DLADCSWHMGELVWCSRVEG |
| 16 | WEADCAWHLGELVWCTPMEF |
| 17 | KEASCSYWLGELVWCDTLTE |
| 18 | KEASCSYWLGELVWCSPGVE |
| 19 | KEASCSYWLGELVWCSGVEG |
| 20 | KEASCSYWLGELVWCSAGVE |
| 21 | ESEDCSYWLGELVWCVAGVE |
| 22 | EKEDCSYWLGTLVWCVAGVE |
| 23 | EDPDCSYWLGELVWCVAGVE |
| 24 | EEADCSYWLGELVWCVAGVE |
| 25 | NADDCSYWLGELVWCVAGVE |
| 26 | SETTCSYWLGELVWCVAGVE |
| 28 | DLADCSYWLGELVWCSRVEG |
| 29 | KEADCAWHLGELVWCVAGVE |
| 30 | KEAECSYHLGELVWCVAGVE |
| 31 | KEARCWYWHGELVWCSDPEE |
| 32 | KEASCSYHLGELVWCVAGVE |
| 33 | KEASCSWHLGELVWCVAGVE |
| 34 | KEASCSYWLGELVWCTEGVE |
| 35 | KEASCSYWLGELVWCDDGVE |
| 36 | KEASCSYWLGELVWCSEGVE |
| 37 | KEASCSYWLGEVWKCKSGVE |
| 38 | KEASCSYWLGELVWCDNGVE |
| 39 | KEASCSYWLGELVWCDTFDE |
| 40 | KEASCSYWLGELVWCDGLDE |
| 41 | KEASCSYWLGELVWCVGLDE |
| 42 | KEASCSYWLGELVWCEDTLE |
| 43 | KEASCSYWLGELVWCEDTME |
| 44 | KEASCSYWLGELVWCEDMME |
| 45 | WVEDCSWHMGELVWCDGGEF |
| 46 | KEASCSYWLGELVWCDWMNG |
| 47 | KEASCSYWLGELVWCDDTPV |
| 48 | KEASCSYWLGELVWCDDYGE |
| 49 | KEASCSYWLGELVWCSDLWE |
| 50 | WRGGCSWHMGELVWCEHDME |
| 51 | AVSKCSFHMGELVWCSDVMN |
| 52 | NQVSCSYSRGELVWCSKQSQ |
| 53 | GRMECAWHQGELVWCTPTLE |
| 54 | GTMECSWHQGELVWCTPTLA |
| 55 | EMRDCSWHLGELVWCAHMEG |
| 56 | GSWECAYHLGELVWCETGSG |
| 57 | VAEPCAYHLGELVWCEVLKG |
| 58 | KEAMCSYWLGELVWCESDMP |
| 59 | DLADCSWHLGELVWCSRVEG |
| 60 | DLADCSWHLGELVWCVGLDE |
| 61 | WVEDCSWHLGELVWCVGLDF |
| 62 | KVADCAWHMGELVWCTEVEG |
| 63 | GEEDCSYHLGELVMCTELDD |
| 64 | GVADCAWHLGELVWCTERED |
| 65 | GEEDCAWHLGELVWCSGGDF |
| 66 | WEADCAWHLGELVWCTKVEE |
| 67 | GEADCSYHLGELVWCNDFEE |
| 68 | WVDCAYHLGELVWCSTFEE |
| 69 | WVEDCAWHMGELVWCTKVDE |
| 70 | READCAWHLGELVWCSERDL |
| 71 | EEASCAYHLGELVWCDAFDV |
| 72 | RVASCAWHLGELVWCDGLDG |
| 73 | GEADCAWHLGELVWCTKVEE |
| 74 | GEASCAYHLGELVWCDEGEG |
| 75 | RVEDCAYHLGLVWCTEGDE |
| 76 | EEPDCSWHLGELVMCTPMEV |
| 77 | KEADCAWHMGELVWCSEMEG |
| 78 | EQADCAWHLGELVWCTPMVF |
| 79 | EEPDCSWHLGELVWCTPIEV |
| 80 | GEPDCAWHLGELVWCTPMVF |
| 81 | GEQDCSYHMGELVWCTTVDG |
| 82 | GVRNCAYHLGELVWCTPMEF |
| 83 | RVADCAWHMGELVWCSELEV |
| 84 | GEADCAWHLGELVWCTPMDL |
| 85 | GEQDCSWHLGELVWCTPMEV |
| 86 | GMRDCSYHLGELVWCSDMEL |
| 87 | EVADCSWHLGELVWCTEGEF |
| 88 | GEEDCAWHLGELVWCTDVED |
| 89 | EVEDCAYHLGELVWCSDLEG |
| 90 | WEEDCAWHLGELVWCAEFDE |
| 91 | KEASCAWHLGELVWCSEVEE |
| 92 | AEADCAWHLGELVMCTKVEE |
| 93 | WAADCAWHLGELVWCTKVEE |
| 94 | WEPDCAWHLGELVWCTKVEE |
| 95 | WEAACAWHLGELVWCTKVEE |
| 96 | WEAACSWHLGELVWCTKVEE |
| 97 | WEADCAAHLGELVWCTKVEE |
| 98 | WEADCAMALGELVWCTKVEE |
| 99' | WEADCAWHAGELVWCTKVEE |
| 100 | WEADCAWHLAELVWCTKVEE |
| 101 | WEADCAWHLGALVWCTKVEE |
| 102 | WEADCAWHLGEAVWCTKVEE |
| 104 | WEADCAWHLGELVACTKVEE |
| 105 | WEADCAWHLGELVWCAKVEE |
| 106 | WEADCAWHLGELVWCTAVEE |
| 107 | WEADCAWHLGELVWCTKAEE |
| 108 | WEADCAWHLGELVWCTKVAE |

10. The peptide ligand according to claim 1 wherein the amino acid sequence is cyclized by the presence of disulfide-bonded Cys residues.

11. The peptide ligand according to claim 1, wherein the peptide ligand is less than 50 amino acid residues.

12. The peptide ligand according to claim 1, wherein the peptide ligand is less than 40 amino acid residues.

13. The peptide ligand according to claim 1, wherein the peptide ligand is between 10 and 30 amino acid residues.

14. The peptide ligand according to claim 1, wherein the peptide ligand has an affinity for IgG-Fc of less than 1 nM.

15. The peptide ligand according to claim 1, wherein the peptide ligand is non-covalently adsorbed or covalently bound to a macromolecule.

16. The peptide ligand according to claim 15, wherein the macromolecules is a solid support.

17. The peptide ligand according to claim 1, wherein the peptide ligand is labelled with a detectable, moiety.

18. A hybrid molecule comprising
the peptide ligand according to any one of claims 1-14; and
a bioactive compound.

19. The hybrid molecule according to claim 18, wherein the bioactive compound is a therapeutic or diagnostic agent.

20. The hybrid molecule according to claim 19, wherein the therapeutic agent is any agent selected from the group consisting of an enzyme, a hormone, a cytokine, an antibody, an antibody fragment, an analgesic, an antipyretic, an antiinflammatory agent, an antibiotic, and antiviral agent, an anti-fungal drug, a cardiovascular drug, a drug that acts on the central nervous system, a drug that affects renal function and electrolyte metabolism and a chemotherapeutic agent.

21. The hybrid molecule according to claim 18 wherein the peptide ligand is DCAWHLGELVWCT (SEQ ID NO: 8) and the therapeutic agent is an anti-tissue factor Fab antibody fragment.

22. A composition comprising the hybrid molecule according to any one of claims 18-21 and a pharmaceutically acceptable carrier.

23. A hybrid molecule according any one of claims 18-21, for use in a method of therapy.

24. A hybrid molecule according any one of claims 18-21, for use in a method of diagnosis.

25. Use of a hybrid molecule according to any one of claims 18-21 in the manufacture of a medicament.

26. Use of a hybrid molecule according to any one of claims 18-21 in the manufacture of a diagnostic agent.

27. A nucleic acid encoding a peptide ligand according to any one of claims 1 to 14.

28. A nucleic acid encoding a hybrid molecule according to any one of claims 18-21.

29. A method of purifying a protein, comprising
culturing a host cell containing a vector comprising a gene fusion, the gene fusion comprising a gene encoding the protein and a gene encoding a peptide ligand according to any one of claims 1 to 14,
resulting in production of a fusion protein comprising the protein and the peptide ligand; and
purifying the fusion protein on a solid IgG-Fc support using the peptide ligand as an affinity handle.

30. A method for purifying IgG, comprising
immobilising a peptide ligand of any one of claims 1 to 14 on a solid support, and
contacting the immobilised peptide ligand with a sample containing the IgG to be purified.

## Patentansprüche

1. Peptidligand, der eine Aminosäuresequenz Xaaⱼ-Cys-Xaaⱼ-CyS-Xₖ umfasst, die IgG-Fc bindet, worin Xaa eine beliebige Aminosäure ist, Xᵢ 0-4 Aminosäuren ist, Xaaⱼ Xaa-Xaa-Xaa-Xaa-Xaa-Xaa-Leu-Val-Trp (Seq.-ID Nr. 9) ist, Xₖ 0-5 Aminosäuren ist.

2. Peptidligand nach Anspruch 1, worin Xaaⱼ Xaa-Xaa-Xaa-Xaa-Gly-Glu-Leu-Val-Trp (Seq.-ID Nr. 10) ist.

3. Peptidligand nach Anspruch 2, worin Xaaⱼ Xaa₁-Xaa₂-Xaa₃-Xaa₄-Gly-Glu-Leu-Val-Trp-Cys ist und
Xaa₁ Ala, Ser oder Thr ist,
Xaa₂ Trp oder Tyr ist,
Xaa₃ His oder Trp ist,
Xaa₄ Leu oder Met ist.

4. Peptidligand nach Anspruch 1 oder Anspruch 2, der die Aminosäuresequenz Xaa-Xaa-Xaa-Xaa-Cys-Xaa-Xaa-Xaa-Xaa-Xaa-Xaa-Leu-Val-Trp-Cys-Xaa-Xaa-Xaa-Xaa-Xaa (Seq.-ID Nr. 11) umfasst, worin Xaa eine beliebige Aminosäure ist.

5. Peptid nach Anspruch 4, das die Aminosäuresequenz Xaa-Xaa-Xaa-Xaa-Cys-Xaa-Xaa-Xaa-Xaa-Gly-Glu-Leu-Val-Trp-Cys-Xaa-Xaa-Xaa-Xaa-Xaa (Seq.-ID Nr. 12) umfasst.

6. Peptid nach Anspruch 5, das die Aminosäuresequenz Xaa₁-Xaa₂-Xaa₃-Xaa₄-Cys-Xaa₅-Xaa₆-Xaa₇-Xaa₈-Gly-Glu-Leu-Val-Trp-Cys-Xaa₉-Xaa₁₀-Xaa₁₁-Xaa₁₂-Xaa₁₃ (Seq.-ID Nr. 13) umfasst, worin
Xaa₅ Ala, Ser oder Thr ist,
Xaa₆ Trp oder Tyr ist,
Xaa₇ His oder Trp ist,
Xaa₈ Leu oder Met ist und
Xaa₁₋₄ und ₉₋₁₃ jeweils eine beliebige Aminosäure sind.

7. Peptid nach Anspruch 6, worin Xaa₄ Ser, Arg oder Asp ist und Xaa₉ Glu, Ser, Thr oder Val ist (Seq.-ID Nr. 14).

8. Peptid nach einem der vorangegangenen Ansprüche, das die Aminosäuresequenz DCAWHLGELVWCT (Seq.-ID Nr. 8) umfasst.

9. Peptidligand nach Anspruch 1, der eine aus folgender Gruppe ausgewählte Aminosäuresequenz umfasst:
| **SEQ.-ID NR.** | **AMINOSÄURESEQUENZ** |
|---|---|
| 15 - | DLADCSWHMGELVWCSRVEG |
| 16 | WEADCAWHLGELVWCTPMEF |
| 17 | KEASCSYWLGELVWCDTLTE |
| 18 | KEASCSYWLGELVWCSPGVE |
| 19 | KEASCSYWLGELVWCSGVEG |
| 20 | KEASCSYWLGELVWCSAGVE |
| 21 | ESEDCSYWLGELVWCVAGVE |
| 22 | EKEDCSYWLGELVWCVAGVE |
| 23 | EDPDCSYWLGELVWCVAGVE |
| 24 | EEADCSYWLGELVWCVAGVE |
| 25 | NADDCSYWLGELVWCVAGVE |
| 26 | SETTCSYWLGELVWCVAGVE |
| 28 | DLADCSYWLGELVWCSRVEG |
| 29 | KEADCAWHLGELVWCVAGVE |
| 30 | KEAECSYHLGELVWCVAGVE |
| 31 | KEARCWYWHGELVWCSDPEE |
| 32 | KEASCSYHLGELVWCVAGVE |
| 33 | KEASCSWHLGELVWCVAGVE |
| 34 | KEASCSYWLGELVWCTEGVE |
| 35 | KEASCSYWLGELVWCDDGVE |
| 36 | KEASCSYWLGELVWCSEGVE |
| 37 | KEASCSYWLGEVWKCKSGVE |
| 38 | KEASCSYWLGELVWCDNGVE |
| 39 | KEASCSYWLGELVWCDTFDE |
| 40 | KEASCSYWLGELVWCDGLDE |
| 41 | KEASCSYWLGELVWCVGLDE |
| 42 | KEASCSYWLGELVWCEDTLE |
| 43 | KEASCSYWLGELVWCEDTME |
| 44 | KEASCSYWLGELVWCEDMME |
| 45 | WVEDCSWHMGELVWCDGGEF |
| 46 | KEASCSYWLGELVWCDWMNG |
| 47 | KEASCSYWLGELVWCDDTPV |
| 48 | KEASCSYWLGELVWCDDYGE |
| 49 | KEASCSYWLGELVWCSDLWE |
| 50 | WRGGCSWHMGELVWCEHDME |
| 51 | AVSKCSFHMGELVWCSDVMN |
| 52 | NQVSCSYSRGELVWCSKQSQ |
| 53 | GRMECAWHQGELVWCTPTLE |
| 54 | GTMECSWHQGELVWCTPTLA |
| 55 | EMRDCSWHLGELVWCAHMEG |
| 56 | GSWECAYHLGELVWCETGSG |
| 57 | VAEPCAYHLGELVWCEVLKG |
| 58 | KEAMCSYWLGELVWCESDMP |
| 59 | DLADCSWHLGELVWCSRVEG |
| 60 | DLADCSWHLGELVWCVGLDE |
| 61 | WVEDCSWHLGELVWCVGLDF |
| 62 | KVADCAWHMGELVWCTEVEG |
| 63 | GEEDCSYHLGELVMCTELDD |
| 64 | GVADCAWHLGELVWCTERED |
| 65 | GEEDCAWHLGELVWCSGGDF |
| 66 | WEADCAWHLGELVWCTKVEE |
| 67 | GEADCSYHLGELVWCNDFEE |
| 68 | WVDCAYHLGELVWCSTFEE |
| 69 | WVEDCAWHMGELVWCTKVDE |
| 70 | READCAWHLGELVWCSERDL |
| 71 | EEASCAYHLGELVWCDAFDV |
| 72 | RVASCAWHLGELVWCDGLDG |
| 73 | GEADCAWHLGELVWCTKVEE |
| 74 | GEASCAYHLGELVWCDEGEG |
| 75 | RVEDCAYHLGELVWCTEGDE |
| 76 | EEPDCSWHLGELVMCTPMEV |
| 77 | KEADCAWHMGELVWCSEMEG |
| 78 | EQADCAWHLGELVWCTPMVF |
| 79 | EEPDCSWHLGELVWCTPIEV |
| 80 | GEPDCAWHLGELVWCTPMVF |
| 81 | GEQDCSYHMGELVWCTTVDG |
| 82 | GVRNCAYHLGELVWCTPMEF |
| 83 | RVADCAWHMGELVWCSELEV |
| 84 | GEADCAWHLGELVWCTPMDL |
| 85 | GEQDCSWHLGELVWCTPMEV |
| 86 | GMRDCSYHLGELVWCSDMEL |
| 87 | EVADCSWHLGELVWCTEGEF |
| 88 | GEEDCAWHLGELVWCTDVED |
| 89 | EVEDCAYHLGELVWCSDLEG |
| 90 | WEEDCAWHLGELVWCAEFDE |
| 91 | KEASCAWHLGELVWCSEVEE |
| 92 | AEADCAWHLGELVWCTKVEE |
| 93 | WAADCAWHLGELVWCTKVEE |
| 94 | WEPDCAWHLGELVWCTKVEE |
| 95 | WEAACAWHLGELVWCTKVEE |
| 96 | WEAACSWHLGELVWCTKVEE |
| 97 | WEADCAAHLGELVWCTKVEE |
| 98 | WEADCAWALGELVWCTKVEE |
| 99 | WEADCAWHAGELVWCTKVEE |
| 100 | WEADCAWHLAELVWCTKVEE |
| 101 | WEADCAWHLGALVWCTKVEE |
| 102 | WEADCAWHLGEAVWCTKVEE |
| 104 | WEADCAWHLGELVACTKVEE |
| 105 | WEADCAWHLGELVMCAKVEE |
| 106 | WEADCAWHLGELVWCTAVEE |
| 107 | WEADCAWHLGELVWCTKAEE |
| 108 | WEADCAWHLGELVWCTKVAE |

10. Peptidligand nach Anspruch 1, worin die Aminosäuresequenz durch die Gegenwart von Disulfid-gebundenen Cys-Resten zyklisiert ist.

11. Peptidligand nach Anspruch 1, worin der Peptidligand weniger als 50 Aminosäurereste umfasst.

12. Peptidligand nach Anspruch 1, worin der Peptidligand weniger als 40 Aminosäurereste umfasst.

13. Peptidligand nach Anspruch 1, worin der Peptidligand zwischen 10 und 30 Aminosäurereste umfasst.

14. Peptidligand nach Anspruch 1, worin der Peptidligand eine Affinität für IgG-Fc von weniger als 1 nM aufweist.

15. Peptidligand nach Anspruch 1, worin der Peptidligand an ein Makromolekül nichtkovalent adsorbiert oder kovalent gebunden ist.

16. Peptidligand nach Anspruch 15, worin das Makromolekül ein fester Träger ist.

17. Peptidligand nach Anspruch 1, worin der Peptidligand mit einer detektierbaren Gruppierung markiert ist.

18. Hybridmolekül, umfassend:
einen Peptidliganden nach einem der Ansprüche 1-14 und
eine bioaktive Verbindung.

19. Hybridmolekül nach Anspruch 18, worin die bioaktive Verbindung ein Therapeutikum oder Diagnostikum ist.

20. Hybridmolekül nach Anspruch 19, worin das Therapeutikum ein beliebiges aus der aus einem Enzym, einem Hormon, einem Cytokin, einem Antikörper, einem Antikörperfragment, einem Analgetikum, einem Antipyretikum, einem Antiphlogistikum, einem Antibiotikum und einem antiviralen Mittel, einem antifungalen Arzneimittel, einem Kardiakum, einem auf das Zentralnervensystem wirkenden Arzneimittel, einem sich auf die Nierenfunktion und den Elektrolythaushalt auswirkenden Arzneimittel und einem Chemotherapeutikum bestehenden Gruppe ausgewähltes Mittel ist.

21. Hybridmolekül nach Anspruch 18, worin der Peptidligand DCAWHLGELVWCT (Seq.-ID Nr. 8) ist und das Therapeutikum ein Antigewebefaktor-Fab-Antikörperfragment ist.

22. Zusammensetzung, die ein Hybridmolekül nach einem der Ansprüche 18-21 und einen pharmazeutisch annehmbaren Träger umfasst.

23. Hybridmolekül nach einem der Ansprüche 18-21 zur Verwendung in einem Therapieverfahren.

24. Hybridmolekül nach einem der Ansprüche 18-21 zur Verwendung in einem Diagnoseverfahren.

25. Verwendung eines Hybridmoleküls nach einem der Ansprüche 18-21 zur Herstellung eines Medikaments.

26. Verwendung eines Hybridmoleküls nach einem der Ansprüche 18-21 zur Herstellung eines Diagnostikums.

27. Nucleinsäure, die für einen Peptidliganden nach einem der Ansprüche 1 bis 14 kodiert.

28. Nucleinsäure, die für ein Hybridmolekül nach einem der Ansprüche 18-21 kodiert.

29. Verfahren zur Reinigung eines Proteins, umfassend:
das Kultivieren einer Wirtszelle, die einen Vektor enthält, der eine Genfusion umfasst, wobei die Genfusion ein für das Protein kodierendes Gen und ein für einen Peptidliganden nach einem der Ansprüche 1 bis 14 kodierendes Gen umfasst, was in der Produktion eines Fusionsproteins resultiert, welches das Protein und den Peptidliganden umfasst; und
das Reinigen des Fusionsproteins auf einem festen IgG-Fc-Träger unter Verwendung des Peptidliganden als Affinitätshenkel.

30. Verfahren zur Reinigung von IgG, umfassend:
das Immobilisieren eines Peptidliganden nach einem der Ansprüche 1 bis 14 auf einem festen Träger; und
das Kontaktieren des immobilisierten Peptidliganden mit einer Probe, die das zu reinigende IgG enthält.

## Revendications

1. Ligand peptidique comprenant une séquence d'aminoacides Xaaᵢ-Cys-Xaaⱼ-Cys-Xₖ qui se lie à IgG-Fc, dans lequel Xaa représente n'importe quel aminoacide, Xᵢ représente 0 à 4 aminoacides, Xaaⱼ représente Xaa-Xaa-Xaa-Xaa-Xaa-Xaa-Leu-Val-Trp (SEQ ID N° 9), Xₖ représente 0 à 5 aminoacides.

2. Ligand peptidique suivant la revendication 1, dans lequel Xaaⱼ représente Xaa-Xaa-Xaa-Xaa-Gly-Glu-Leu-Val-Trp (SEQ ID N° 10).

3. Ligand peptidique suivant la revendication 2, dans lequel Xaaⱼ représente Xaa₁-Xaa₂-Xaa₃-Xaa₄-Gly-Glu-Leu-Val-Trp-Cys et
Xaa₁ représente Ala, Ser ou Thr,
Xaa₂ représente Trp ou Tyr,
Xaa₃ représente His ou Trp,
Xaa₄ représente Leu ou Met.

4. Ligand peptidique suivant la revendication 1 ou la revendication 2, comprenant la séquence d'aminoacides Xaa-Xaa-Xaa-Xaa-Cys-Xaa-Xaa-Xaa-Xaa-Xaa-Xaa-Leu-Val-Trp-Cys-Xaa-Xaa-Xaa-Xaa-Xaa (SEQ ID N° 11), dans laquelle Xaa représente n'importe quel aminoacide.

5. Peptide suivant la revendication 4, comprenant la séquence d'aminoacides Xaa-Xaa-Xaa-Xaa-Cys-Xaa-Xaa-Xaa-Xaa-Gly-Glu-Leu-Val-Trp-Cys-Xaa-Xaa-Xaa-Xaa-Xaa (SEQ ID N° 12).

6. Peptide suivant la revendication 5, comprenant la séquence d'aminoacides Xaa₁-Xaa₂-Xaa₃-Xaa₄-Cys-Xaa₅-Xaa₆-Xaa₇-Xaa₈-Gly-Glu-Leu-Val-Trp-Cys-Xaa₉-Xaa₁₀-Xaa₁₁-Xaa₁₂-Xaa₁₃ (SEQ ID N° 13), dans laquelle
Xaa₅ représente Ala, Ser ou Thr,
Xaa₆ représente Trp ou Tyr,
Xaa₇ représente His ou Trp,
Xaa₈ représente Leu ou Met, et
Xaa₁₋₄ et ₉₋₁₃ représentent chacun un aminoacide.

7. Peptide suivant la revendication 6, dans lequel Xaa₄ représente Ser, Arg ou Asp et Xaa₉ représente Glu, Ser, Thr ou Val (SEQ ID N° 14).

8. Peptide suivant l'une quelconque des revendications précédentes, comprenant la séquence d'aminoacides DCAWHLGELVWCT (SEQ ID N° 8).

9. Ligand peptidique suivant la revendication 1, comprenant une séquence d'aminoacides choisie dans le groupe consistant en :
| **SEQ ID NO:** | **SEQUENCE D'AMINOACIDES** |
|---|---|
| 15 | DLADCSWHMGELVMCSRVEG |
| 16 | WEADCAWHLGELVWCTPMEF |
| 17 | KEASCSYWLGELVWCDTLTE |
| 18 | KEASCSYWLGELVWCSPGVE |
| 19 | KEASCSYWLGELVWCSGVEG |
| 20 | KEASCSYWLGELVWCSAGVE |
| 21 | ESEDCSYWLGELVWCVAGVE |
| 22 | EKEDCSYWLGELVWCVAGVE |
| 23 | EDPDCSYWLGELVWCVAGVE |
| 24 | EEADCSYWLGELVWCVAGVE |
| 25 | NADDCSYWLGELVWCVAGVE |
| 26 | SETTCSYWLGELVWCVAGVE |
| 28 | DLADCSYWLGELVWCSRVEG |
| 29 | KEADCAWHLGELVWCAGVE |
| 30 | KEAECSYHLGELVWCVAGVE |
| 31 | KEARCWYWHGELVWCSDPEE |
| 32 | KEASCSYHLGELVWCVAGVE |
| 33 | KEASCSWHLGELVWCVAGVE |
| 34 | KEASCSYWLGELVWCTEGVE |
| 35 | KEASCSYWLGELVWCDDGVE |
| 36 | KEASCSYWLGELVWCSEGVE |
| 37 | KEASCSYWLGEVWKCKSGVE |
| 38 | KEASCSYWLGELVWCDNGVE |
| 39 | KEASCSYWLGELVWCDTFDE |
| 40 | KEASCSYWLGELVWCDGLDE |
| 41 | KEASCSYWLGELVWCVGLDE |
| 42 | KEASCSXWLGELVWCEDTLE |
| 43 | KFASCSYWLGELVWCEDTME |
| 44 | KEASCSYWLGELVWCEDMME |
| 45 | WVEDCSWHMGELVWCDGGEF |
| 46 | KEASCSYWLGELVWCDWMNG |
| 47 | KEASCSYWLGELVWCDDTPV |
| 48 | KEASCSYWLGELVWCDDYGE |
| 49 | KEASCSYWLGELVWCSDLWE |
| 50 | WRGGCSWHMGELVWCEHDME |
| 51 | AVSKCSFHMGELVWCSDVMN |
| 52 | NQVSCSYSRGELVWCSKQSQ |
| 53 | GRMECAWHQGELVWCTPTLE |
| 54 | GTMECSWHQGELVWCTPTLA |
| 55 | EMRDCSWHLGELVWCAHMEG |
| 56 | GSWECAYHLGELVWCETGSG |
| 57 | VAEPCAYHLGELVWCEVLKG |
| 58 | KEAMCSYWLGELVMCESDMP |
| 59 | DLADCSWHLGELVWCSRVEG |
| 60 | DLADCSMHLGELVWCVGLDE |
| 61 | WNEDCSWHLGELVWCVGLDF |
| 62 | KVADCAWHMGELVWCTEVEG |
| 63 | GEEDCSYHLGELVMCTELDD |
| 64 | GVADCAWHLGELVWCTERED |
| 65 | GEEDCAWHLGELVWCSGGDF |
| 66 | WEADCAWHLGELVMCTKVEE |
| 67 | GEADCSYHLGELVWCNDFEE |
| 68 | WVDCAYHLGELVWCSTFEE |
| 69 | WVEDCAWHMGELVWCTKVDE |
| 70 | READCAWHLGELVWCSERDL |
| 71 | EEASCAYHLGELVWCDAFDV |
| 72 | RVASCAWHLGELVWCDGLDG |
| 73 | GEADCAWHLGELVWCTKVEE |
| 74 | GEASCAYHLGELVWCDEGEG |
| 75 | RVEDCAYHLGELVWCTEGDE |
| 76 | EEPDCSWHLGELVMCTPMEV |
| 77 | KEADCAWHMGELVWCSEMEG |
| 78 | EQADCAWHLGELVWCTPMVF |
| 79 | ESPPCSMHLGELVMCTPIEV |
| 80 | GEPDCAWHLGELVWCTPMVF |
| 81 | GEQQCSYHMGELVWCTTVDG |
| 82 | GVRNCAYHLGELVWCTPMEF |
| 83 | RVADCAWHMGELVWCSELEV |
| 84 | GEADCAWHLGELVWCTPMDL |
| 85 | GEQDCSWHWELVWCTPMEV |
| 86 | GMRDCSYHLGELVWCSDMEL |
| 87 | EVADCSWHLGELVWCTEGEF |
| 88 | GEEDCAWHLGELVWCTDVED |
| 89 | EVEDCAYHLGELVWCSDLEG |
| 90 | MEEDCAHHLGELVWCAEFDE |
| 91 | KEASCAWHLGELVWCSEVEE |
| 92 | AEADCAWHIABLVWCTKVEE |
| 93 | WAADCAWHLGELVWCTKVEE |
| 94 | WEPDCAWHLGELVWCTKVEE |
| 95 | WEAACAWHLGSLVWCTKVEE |
| 96 | WEAACSWHLGELVWCTKVEE |
| 97 | WEADCAAHLGELVWCTKVEE |
| 98 | WEADCAMALGELVMCTKVEE |
| 99 | WEADCAMHAGELVWCTKVBE |
| 100 | WEADCAWHLAELVWCTKVEE |
| 101 | WEADCAWHLGALVMCTKVEE |
| 102 | WEADCAWHLGEAVWCTKVEE |
| 104 | WEADCAWHLGELVACTKVEE |
| 105 | WEADCAWHLGELVWCAKVEE |
| 106 | WEADCAWHLGELVWCTAVEE |
| 107 | WEADCAWHLGELVWCTKAEE |
| 108 | WEADCAWHLGELVWCTKVAE |

10. Ligand peptidique suivant la revendication 1, dans lequel la séquence d'aminoacides est cyclisée par la présence de résidus Cys liés par des liaisons disulfure.

11. Ligand peptidique suivant la revendication 1, ledit ligand peptidique comprenant moins de 50 résidus d'aminoacides.

12. Ligand peptidique suivant la revendication 1, ledit ligand peptidique comprenant moins de 40 résidus d'aminoacides.

13. Ligand peptidique suivant la revendication 1, ledit ligand peptidique comprenant 10 à 30 résidus d'aminoacides.

14. Ligand peptidique suivant la revendication 1, ledit ligand peptidique ayant une affinité pour IgG-Fc inférieure à 1 nM.

15. Ligand peptidique suivant la revendication 1, ledit ligand peptidique étant adsorbé de manière non covalente ou lié par covalence à une macromolécule.

16. Ligand peptidique suivant la revendication 15, la macromolécule étant un support solide.

17. Ligand peptidique suivant la revendication 1, ledit ligand peptidique étant marqué avec un groupe détectable.

18. Molécule hybride comprenant
le ligand peptidique suivant l'une quelconque des revendications 1 à 14 ; et
un composé biologiquement actif.

19. Molécule hybride suivant la revendication 18, dans laquelle le composé biologiquement actif est un agent thérapeutique ou agent de diagnostic.

20. Molécule hybride suivant la revendication 19, dans laquelle l'agent thérapeutique est n'importe quel agent choisi dans le groupe consistant en une enzyme, une hormone, une cytokine, un anticorps, un fragment d'anticorps, un analgésique, un antipyrétique, un agent anti-inflammatoire un antibiotique, un agent antiviral, un médicament antifongique, un médicament cardio-vasculaire, un médicament qui agit sur le système nerveux central, un médicament qui a un effet sur la fonction rénale et le métabolisme des électrolytes et un agent chimiothérapeutique.

21. Molécule hybride suivant la revendication 18, dans laquelle le ligand peptidique est DCAWHLGELVWCT (SEQ ID N° 8) et l'agent thérapeutique est un fragment d'anticorps Fab anti-facteur tissulaire.

22. Composition comprenant la molécule hybride suivant l'une quelconque des revendications 18 à 21 et un support pharmaceutiquement acceptable.

23. Molécule hybride suivant l'une quelconque des revendications 18 à 21, destinée à être utilisée dans une méthode de thérapie.

24. Molécule hybride suivant l'une quelconque des revendications 18 à 21, destinée à être utilisée dans une méthode de diagnostic.

25. Utilisation d'une molécule hybride suivant l'une quelconque des revendications 18 à 21 dans la production d'un médicament.

26. Utilisation d'une molécule hybride suivant l'une quelconque des revendications 18 à 21 dans la production d'un agent de diagnostic.

27. Acide nucléique codant pour un ligand peptidique suivant l'une quelconque des revendications 1 à 14.

28. Acide nucléique codant pour une molécule hybride suivant l'une quelconque des revendications 18 à 21.

29. Procédé pour purifier une protéine, comprenant
la culture d'une cellule hôte contenant un vecteur comprenant une fusion de gènes, la fusion de gènes comprenant un gène codant pour la protéine et un gène codant pour un ligand peptidique suivant l'une quelconque des revendications 1 à 14, avec pour résultat la production d'une protéine de fusion comprenant la protéine et le ligand peptidique ; et
la purification de la protéine de fusion sur un support IgG-Fc solide en utilisant le ligand peptidique comme manche d'affinité.

30. Procédé pour purifier une IgG, comprenant
l'immobilisation d'un ligand peptidique de l'une quelconque des revendications 1 à 14 sur un support solide ; et
la mise en contact du ligand peptidique immobilisé avec un échantillon contenant la IgG à purifier.
